# EUROPEAN PATENT SPECIFICATION

(11) **EP 2 219 452 B1**
(45) Date of publication and mention of the grant of the patent: **14.10.2015**
(21) Application number: 08847680.9
(22) Date of filing: 05.11.2008
(51) Int. Cl.: A01N 43/54, A61K 39/395, C07K 16/24

(54) **METHODS OF TREATING SCLERODERMA**
VERFAHREN ZUR BEHANDLUNG VON SKLERODERM
PROCÉDÉS DE TRAITEMENT DE LA SCLÉRODERMIE

(30) Priority: 05.11.2007 US 996175 P; 26.09.2008 US 100454 P
(43) Date of publication of application: 25.08.2010
(73) Proprietor: MedImmune, LLC, Gaithersburg, MD 20878 (US)
(72) Inventor: COYLE, Anthony, Washington, DC 20037 (US)
(74) Representative: Winter, Christopher Spencer
(86) International application number: PCT/US2008/082481
(87) International publication number: WO 2009/061818

(56) References cited:
- WO-A1-2005/067963
- WO-A2-2007/002096
- US-A1- 2003 088 884
- US-A1- 2004 265 305
- US-A1- 2007 092 890
- US-A1- 2007 092 890
- US-B1- 6 333 032
- MACDERMOTT EMMA JANE ET AL: "Activation of type I interferon pathway in patients with systemic sclerosis (SSc) is associated with diffuse disease, antibodies to Ro and absence of Caucasian race", ARTHRITIS & RHEUMATISM, vol. 56, no. 12, December 2007 (2007-12), page 4244, XP009157116, & 71ST ANNUAL MEETING OF THE AMERICAN-COLLEGE-OF-RHEUMATOLOGY; BOSTON, MA, USA; NOVEMBER 06 -11, 2007 ISSN: 0004-3591

## Description

This application claims priority to U.S. Patent Application Nos. 60/996,175 and 61/100,545.

### FIELD OF THE INVENTION

The present invention relates to an antagonist of type I interferon (IFN), and compositions thereof, for use in methods of treating/ameliorating scleroderma and associated symptoms, wherein the antagonist is an anti-IFNαR antibody or an anti-IFNα antibody.

### BACKGROUND OF THE INVENTION

Scleroderma, or systemic sclerosis (SSC), is a progressive, debilitating autoimmune disorder characterized by excess protein deposition into the extracellular matrix by dermal fibroblasts, also referred to as dermal fibrosis. Patients with diffuse cutaneous disease often present unique markers such as upregulation of type I interferon (IFN)-induced genes in skin as well as serum antinuclear autoantibodies specific for topoisomerase I. Supporting the idea that IFN plays a role in dermal fibrosis are recent reports of scleroderma arising in patients receiving IFN therapy for chronic viral infection. For a review of systemic sclerosis, see Varga & Abraham, 2007, J. Clin. Invest., 117:557-567.

Type I IFNs, α, β, θ, κ, and ω, are cytokines expressed from 13 functional IFN-α genes, one IFN-β gene, one IFN-θ gene, one IFN- κ gene, and one IFN-ω gene. (Theofilopoulos AN, Baccala R, Beutler B, Kono DH. Type I Interferons (α/β) in immunity and autoimmunity. Immunol Rev. 2005 Apr; 204:9-26). There are at least 28 potential IFN-α subtypes, with the following being a partial listing of these: α1, α2a, α2b, α4, α5, α6, α7, α8, α10, α16, α17, and α21. In certain instances, reference to interferon alpha subtype α2 encompasses both α2a and α2b.

All human type I interferons bind to a cell surface receptor (IFN alpha receptor, IFNAR) consisting of two transmembrane proteins, IFNAR-1 and IFNAR-2 (Uze et al. (1990) Cell 60:225; Novick et al. (1994) Cell 77:391). Binding to this receptor results in the activation of intracellular signal transduction pathways (Stark GR, Kerr IM, Williams BR, Silverman RH, Schreiber RD. Annu Rev Biochem 1998; 67:227-64), initiated by the activation of the Jak kinases, Jak1 and Tyk2. These kinases subsequently phosphorylate signal transducer and activator of transcription (STAT) proteins, STATs 1 and 2. Phosphorylated STAT proteins form the transcription factor complex, IFN-stimulated gene factor 3 (ISGF3) that, together with p48, translocates into the nucleus. These complexes activate the IFN-stimulated response element (ISRE) that induces the expression of IFN-inducible genes.

In addition to specific antiviral functions, Type I IFNs play a critical role in the regulation of the immune system. (Theofilopoulos AN, Baccala R, Beutler B, Kono DH. Type I Interferons (a/b) in immunity and autoimmunity. Immunol Rev. 2005 Apr; 204:9-26 and Belardelli F, Gresser I. The neglected role of type I interferon in the T-cell response: implications for its clinical use. Immunol Today 1996; 17:369-72). Various types of cells including monocytes, macrophages, DCs, and lymphocytes, as well as other hematological cells, produce Type I IFNs in response to pro-inflammatory cytokines, as well as components of various pathogens. These cells also respond to Type I IFN and enhance the expression of immunologically important molecules such as MHC class I, CD38, interleukins (BLyS, IL-6, IL-10 and IL-15), and chemokines (IL-8, MCP-1, MCP-2, MIG, MIP1a, MIP1b, and IP10). Moreover, type I IFNs induce multiple biological functions in key components of the immune system including dendritic, T, B, and natural killer (NK) cells. For example, Type I IFNs promote DC maturation, memory CD8+ T cell proliferation, inhibition of CD4+ T cell apoptosis, NK cell activation, and B cell differentiation. (Banchereau J, Pascual V, Palucka AK. Autoimmunity through cytokine-induced dendritic cell activiation. Immunity, Vol. 20, 539-550, May, 2004 and Taki S. Cytokine & Growth Factor Reviews 13 (2002) 379-391 and Mailliard RB, Son YI, Redlinger R, Coates PT, Giermasz A, Morel PA, Storkus WJ, Kalinski P. J Immunol. 2003 Sep 1; 171(5):2366-73).

While almost all cells can produce Type I IFNs in response to stimulation by viral and bacterial components, plasmacytoid dendritic cells (pDCs), or "natural IFN-producing cells" produce up to 1000-fold more Type I IFN than other cell types. Type I IFN production can be induced by the stimulation of endosomal Toll-Like Receptors (TLR), such as TLR7 and TLR9, with single stranded RNA (ssRNA), hypomethylated CpGs in bacterial DNA, or autoantigen-antibody immune complexes.

There is a need for a better understanding of the role of type I interferons in the pathogenesis scleroderma and for the identification of new treatments for this disease and its clinical manifestations.

### SUMMARY OF THE INVENTION

The present invention relates to an antagonist of type I IFN, and compositions thereof, for use in methods of treating scleroderma and the symptoms associated with scleroderma in a patient in need of such treatment. The present disclosure further provides an antagonist of type I IFN, and compositions thereof, for use in methods of reducing type I IFN-inducible gene expression associated with scleroderma. The antagonist of type I IFN of the invention is an anti-IFNαR antibody or an anti-IFNα antibody.

### BRIEF DESCRIPTION OF THE FIGURES

For the purpose of illustrating the invention, there are depicted in the drawings certain embodiments on the invention. However, the invention is not limited to the precise arrangements and instrumentalities of the embodiments depicted in the drawings.

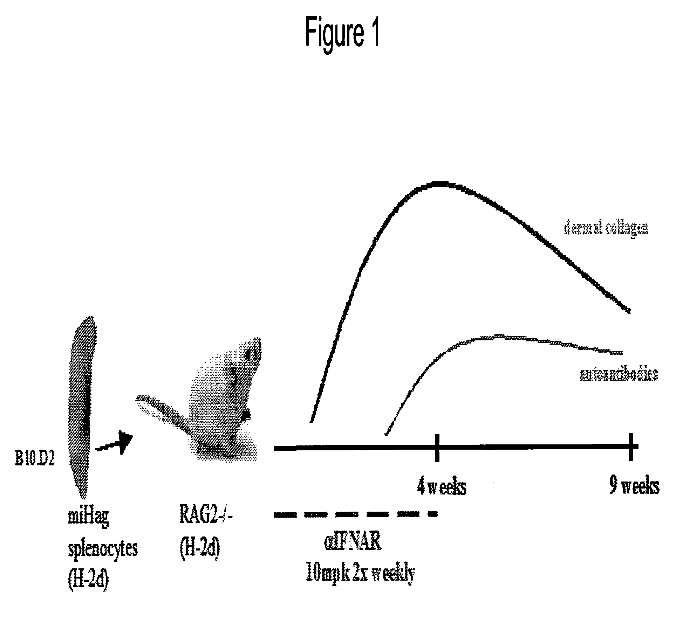

**Figure 1** diagrams the production of a model of systemic sclerosis (SSc) in mice, *i.e.*, disease induction, wherein RAG2-/- mice are grafted with minor histocompatibility (miHag)-mismatched total splenocytes, and SSc symptoms such as dermal collagen deposition and autoantibodies develop over time.

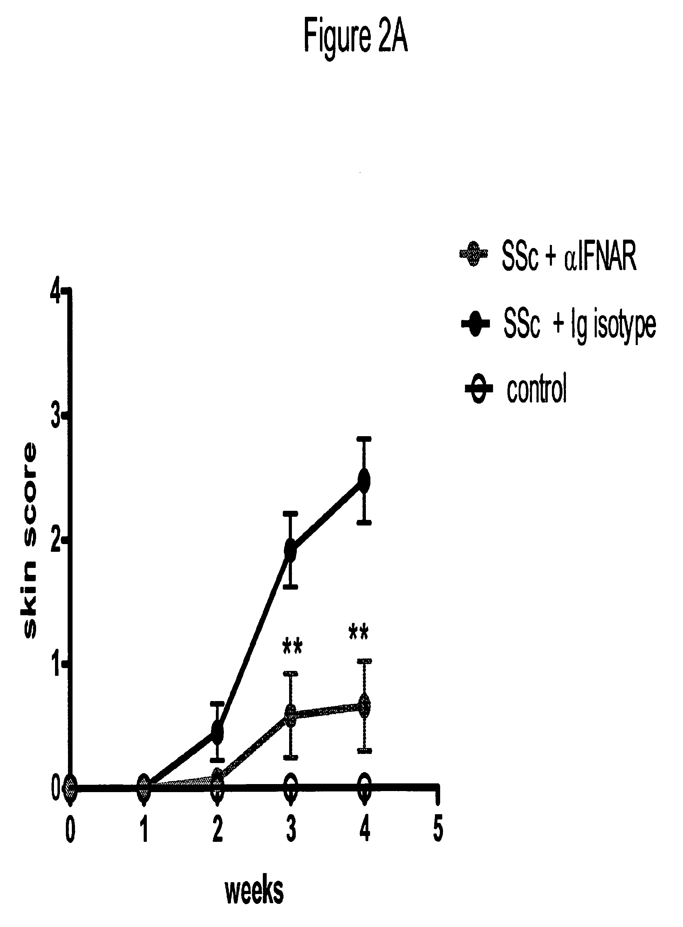

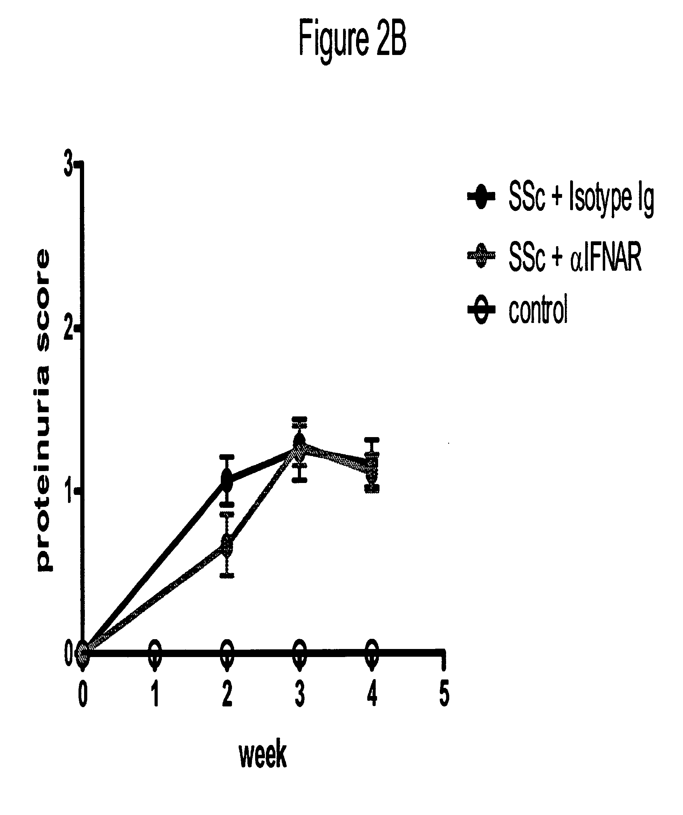

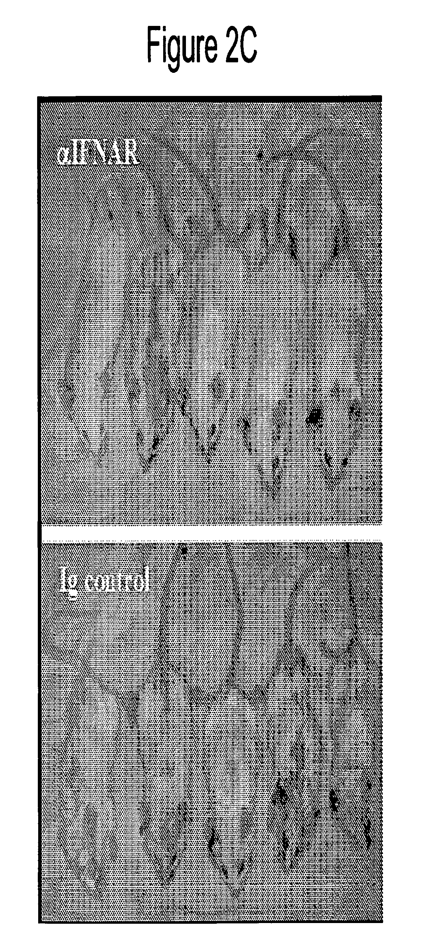

**Figure 2A** **and** **B** are graphs of the clinical signs of scleroderma measured over time in mice having no disease induction (control), having disease induction in the presence of an anti-interferon alpha receptor (IFNAR) antibody (SSc + αIFNAR), or having disease induction in the presence of an Ig isotype control antibody (SSc + Ig Isotype; Fig. 2A) or (SSc + Isotype Ig; Fig. 2B). Shown on the Y-axes are the skin score (Fig. 2A) and the proteinuria score (Fig. 2B). Figure 2C presents photos of mice following disease induction in the presence of an anti-interferon alpha receptor antibody (αIFNAR; top panel), or in the presence of an Ig isotype control antibody (Ig Control; top panel).

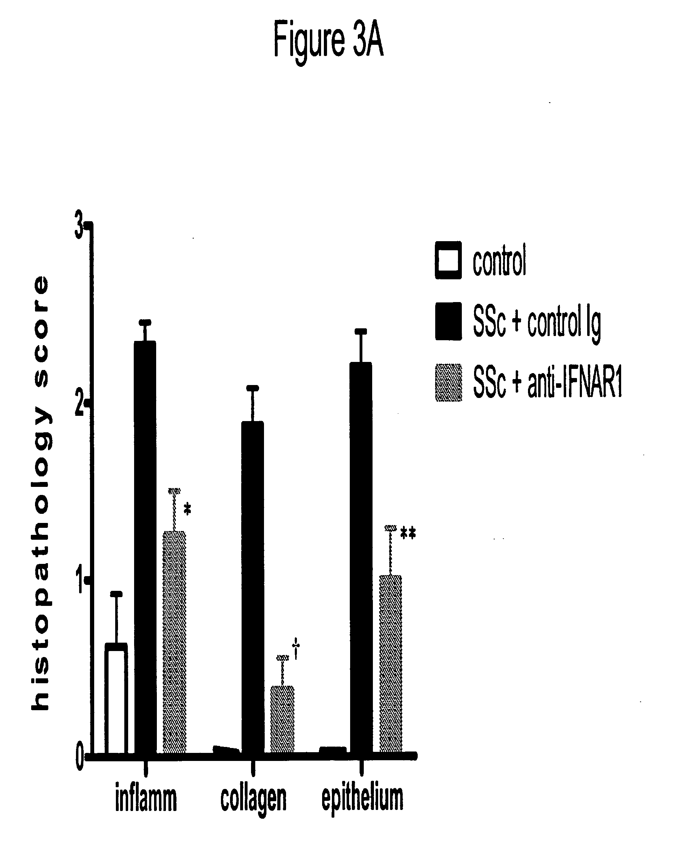

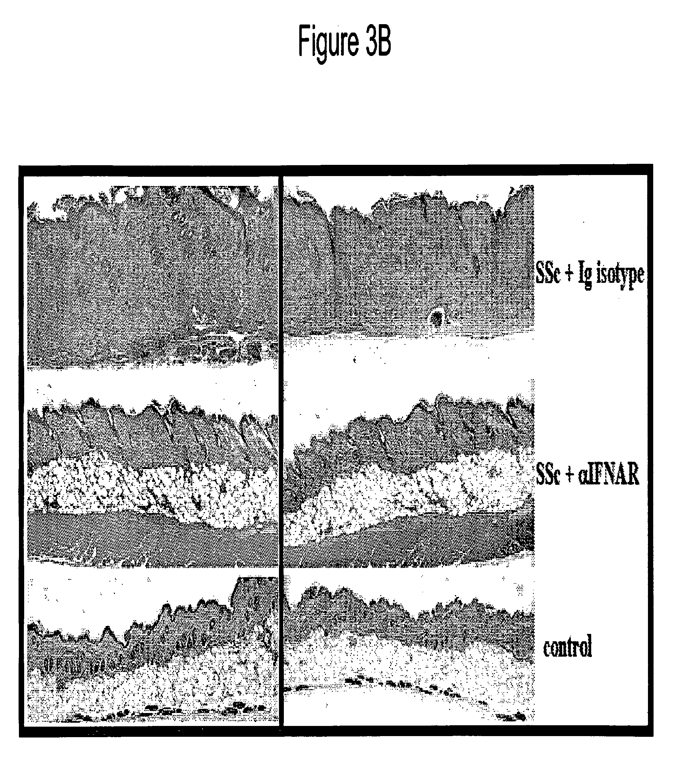

**Figure 3A** is a bar graph depicting the histopathological analysis of SSc skin from RAG2-/-mice having no disease induction (no graft) (control), having disease induction in the presence of an anti-interferon alpha receptor antibody (SSc + αIFNAR), or having disease induction in the presence of an Ig isotype control antibody (SSc + Ig Isotype). Shown on the Y-axis are the additive scores for inflammation (0 = normal; 1= sparse cellular infiltrate; 2 = moderate infiltrate; 3 = pervasive dermal infiltrate) and collagen deposition (0 = normal, 1 = mild; 2 = moderate; 3 = severe). Figure 3B shows immunohistochemistry of representative H&E (left panel) and Masson's trichrome (right panel) stains of skin from mice having no disease induction (control), having disease induction in the presence of an anti-interferon alpha receptor antibody (SSc + αIFNAR), or having disease induction in the presence of an Ig isotype control antibody (SSc + Ig Isotype).

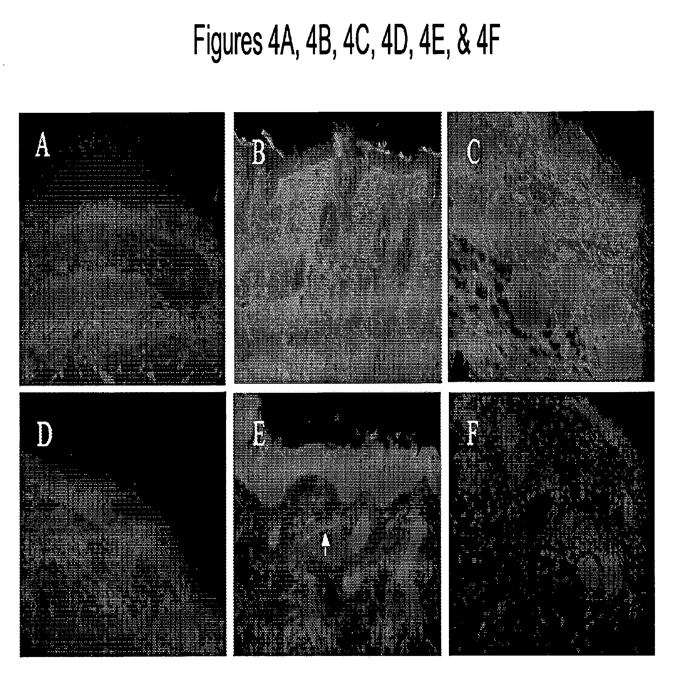

**Figures 4A - 4F** show immunohistochemistry of skin sections stained with either a goat anti-mouse Ig-FITC (green) or rat anti-mouse C1q-PE polyclonal antibody (red) and mounted in DAPI (blue). Skin sections came from syngeneic graft control mice (Figs. 4A and 4D), mice with SSc induction in the presence of Ig isotype control (Figs. 4B and 4E), and mice with SSc induction in the presence of anti-IFNAR antibody (Figs. 4C and 4F).

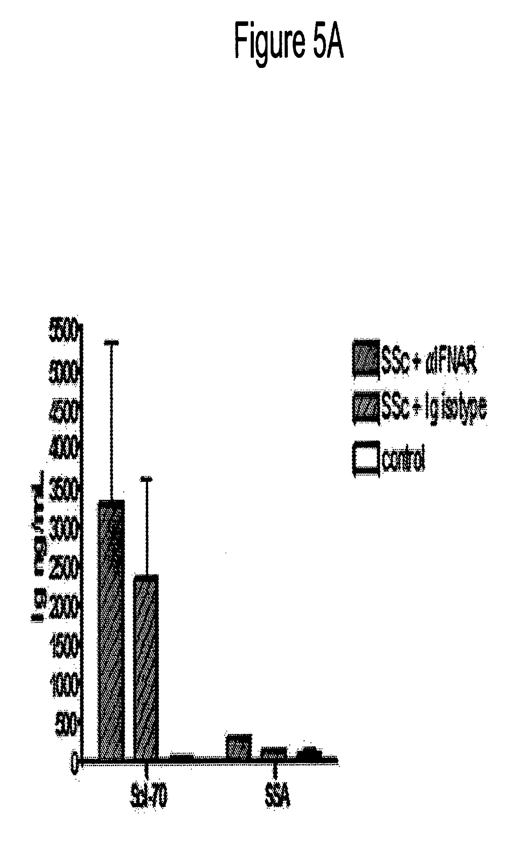

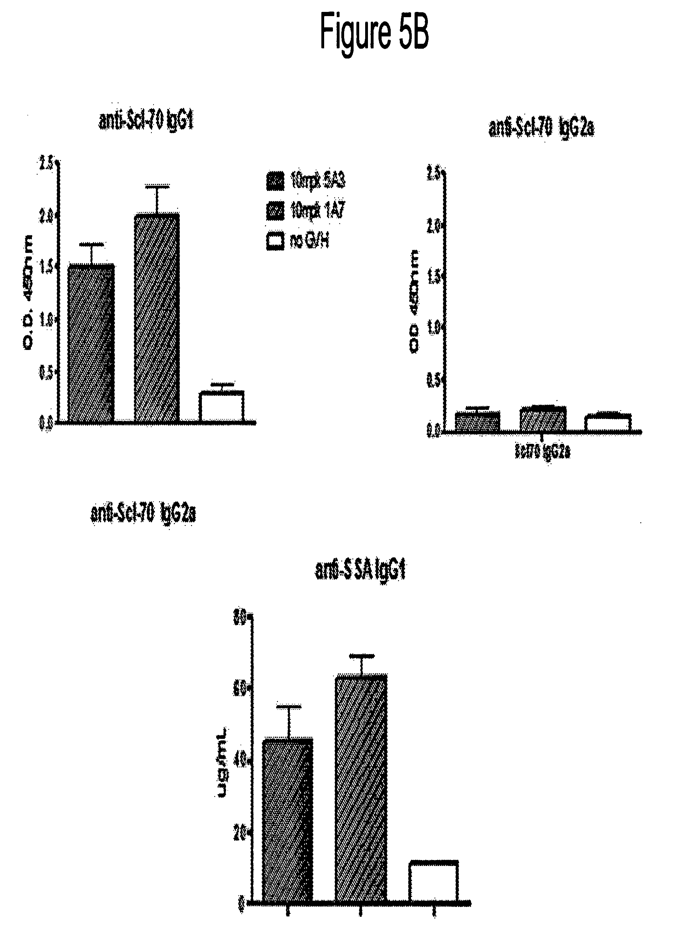

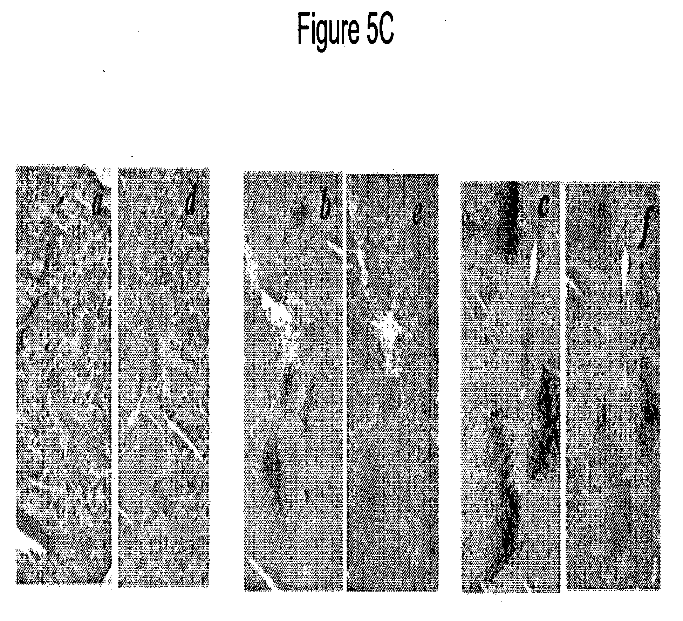

**Figure 5A** is a bar graph depicting serum anti-Scl-70 and anti-SSA autoantibodies (IgG, IgA, IgM) as detected by ELISA of sera from mice having no disease induction (control), having disease induction in the presence of an anti-interferon alpha receptor antibody (SSc + αIFNAR), or having disease induction in the presence of an Ig isotype control antibody (SSc + Ig Isotype). Figure 5B presents 3 bar graphs depicting the amounts of anti-Scl-70 IgG1 (top), anti-Scl-70 IgG2 (middle), and anti-SSA IgG1 (bottom), in sera from mice having no disease induction (no GVH), having disease induction in the presence of an anti-interferon alpha receptor antibody (10 mpk A53), or having disease induction in the presence of an Ig isotype control antibody (10 mpk 1A7). Figure 5C shows immunohistochemisty of spleen sections from syngeneic graft control mice (panels a and d), mice with SSc induction in the presence of Ig isotype control (panels b and e), and mice with SSc induction in the presence of anti-IFNAR antibody (panels c and f), stained for CD45R/B220 (brown), and peanut agglutinin (red) to identify germinal centers (GC).

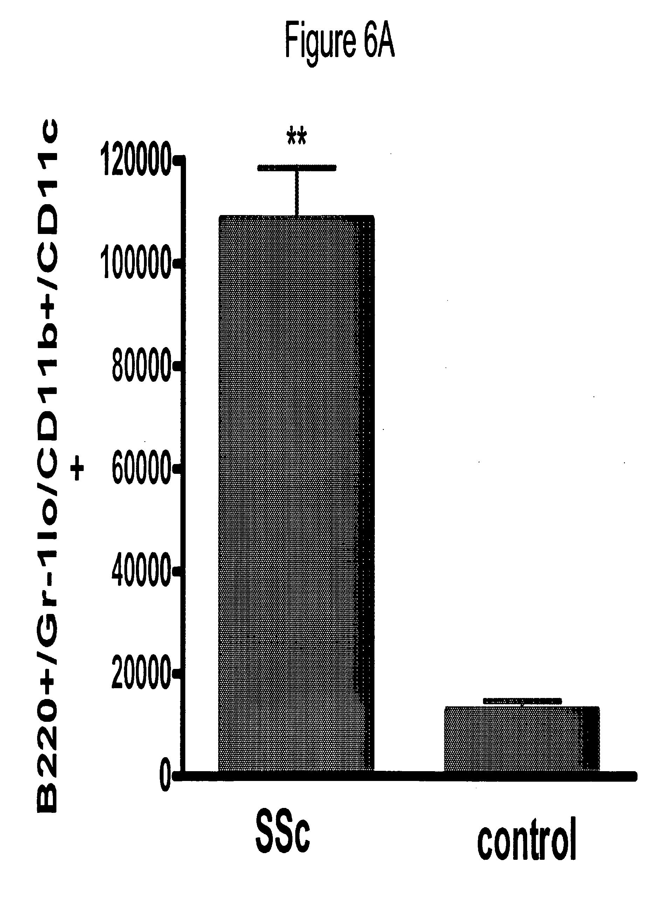

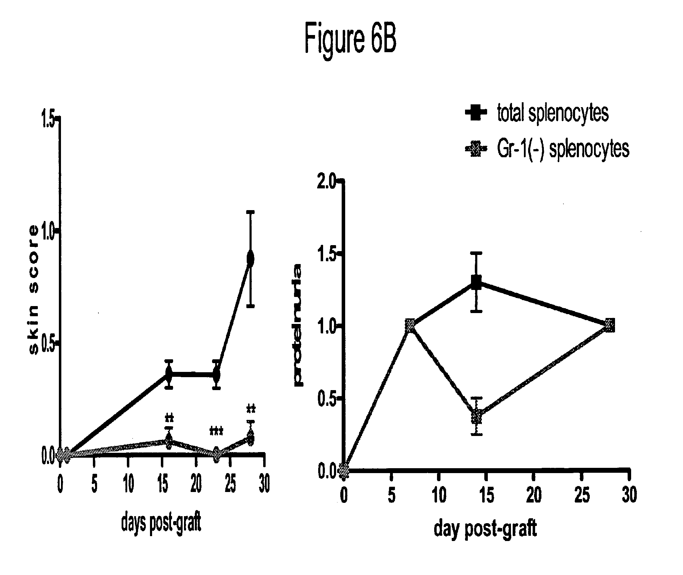

**Figure 6A** is a bar graph depicting the quantitative analysis by flow cytometry cell sorting (FACS) of the number of splenic plasmacytoid dendritic cell (pDC) (B220+/ Gr-1lo /CD11c+/CD11b-) in mismatch graft recipients 2 weeks post-graft (SSc) or ungrafted RAG2-/- controls (control). Figure 6B presents time course graphs of skin score (left) and proteinuria (right) in RAG2-/- mice that were grafted with total miHag mismatched splenocytes (total splenocytes) or with Gr-1-depleted (*i.e.,* pDC-depleted) miHag mismatched splenocytes (Gr-1(-) splenocytes).

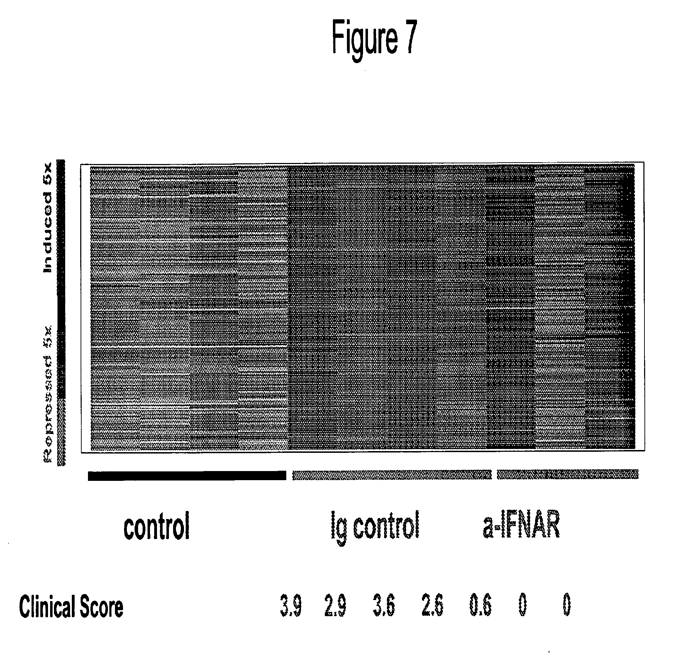

**Figure 7** is a "heatmap" presentation of whole genome array (WGA) data analysis of genes that are repressed or induced in the skin of mice having no SSc disease induction (control), having SSc disease induction in the presence of an Ig isotype control antibody (Ig control), or having SSc disease induction in the presence of an anti-interferon alpha receptor antibody (a-IFNAR). Clinical skin scores, determined as described for Figure 3A, are shown below the columns for each WGA analyzed sample.

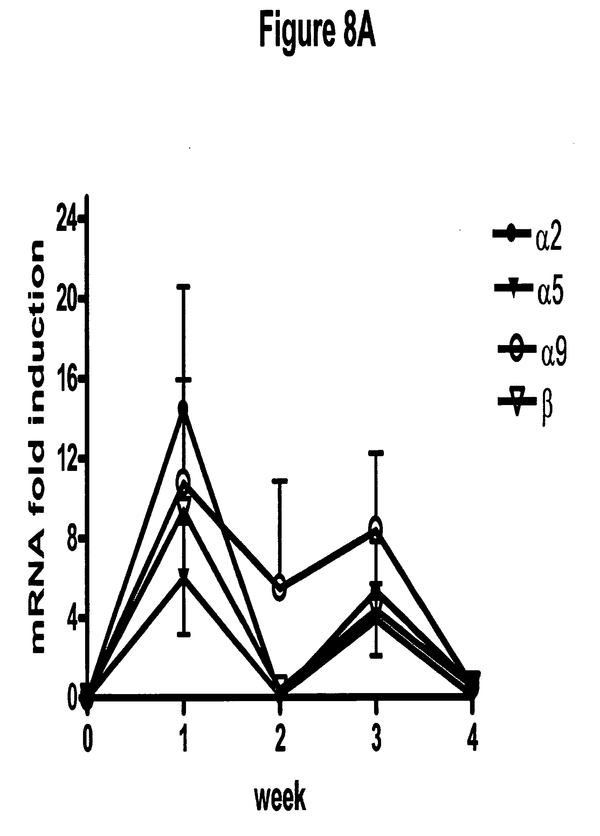

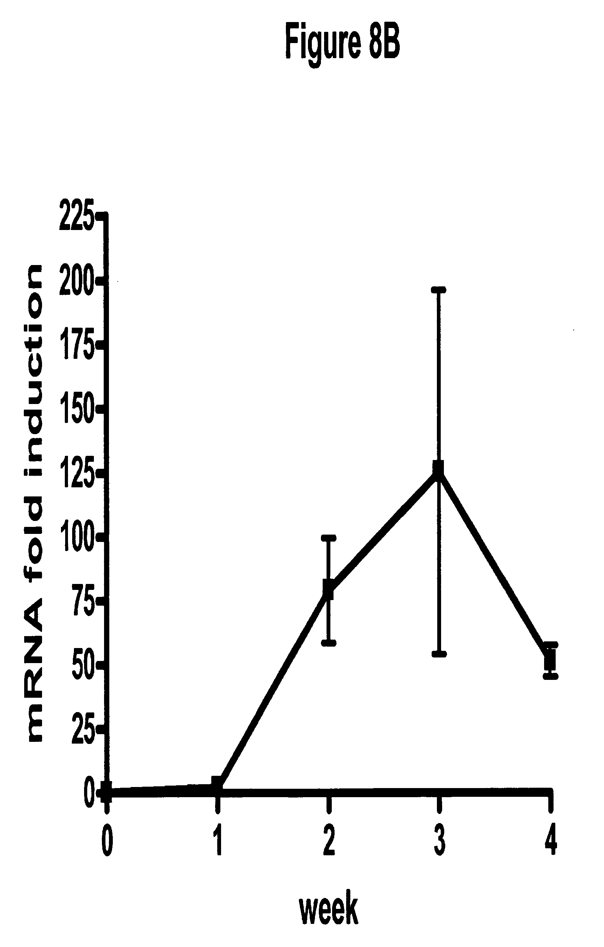

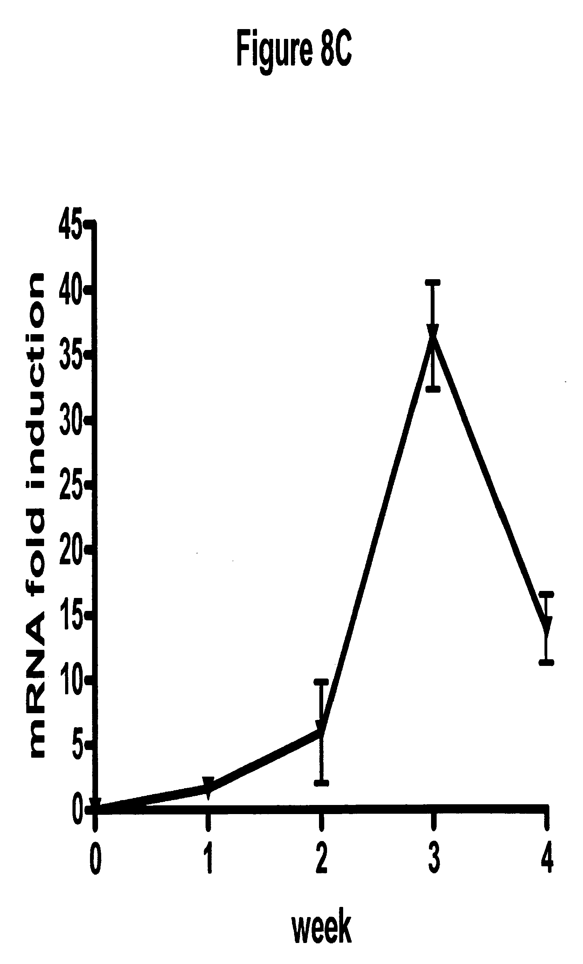

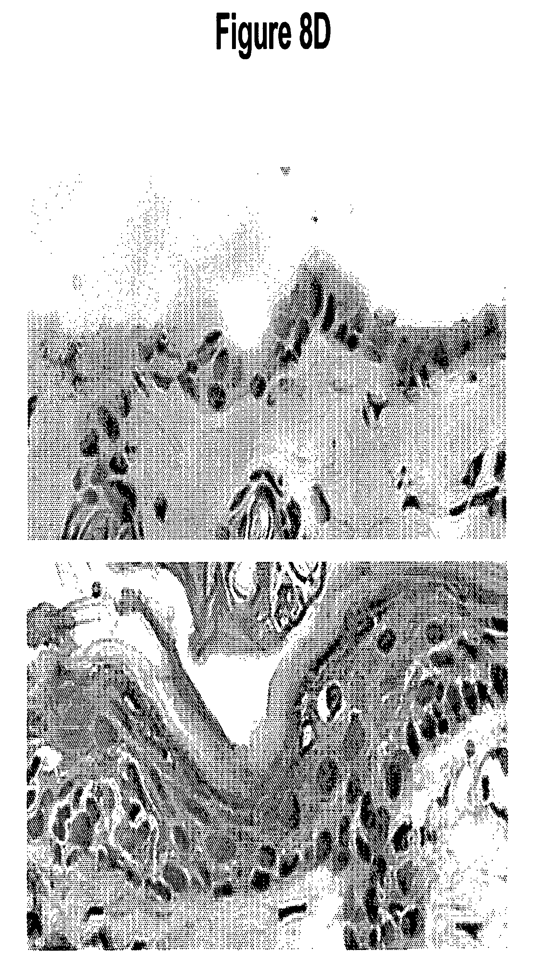

**Figures 8A - 8D** summarize a series of experiments directed to analyzing temporal expression of type I IFNs in GVH-SSc skin. Figure 8A summarizes qPCR analysis of IFNα2, α5, α9, and β mRNA induction. Figures 8B and 8C summarize qPCR analysis of IFNγ and IFNλ-2, respectively (data are representative of 2 studies, n=4/timepoint). Figure 8D depicts immunohistochemical staining of IFNλ-3 in GVH-SSc (bottom panel) and non-SSc (top panel) dermal epithelial cells (magnification, 400x).

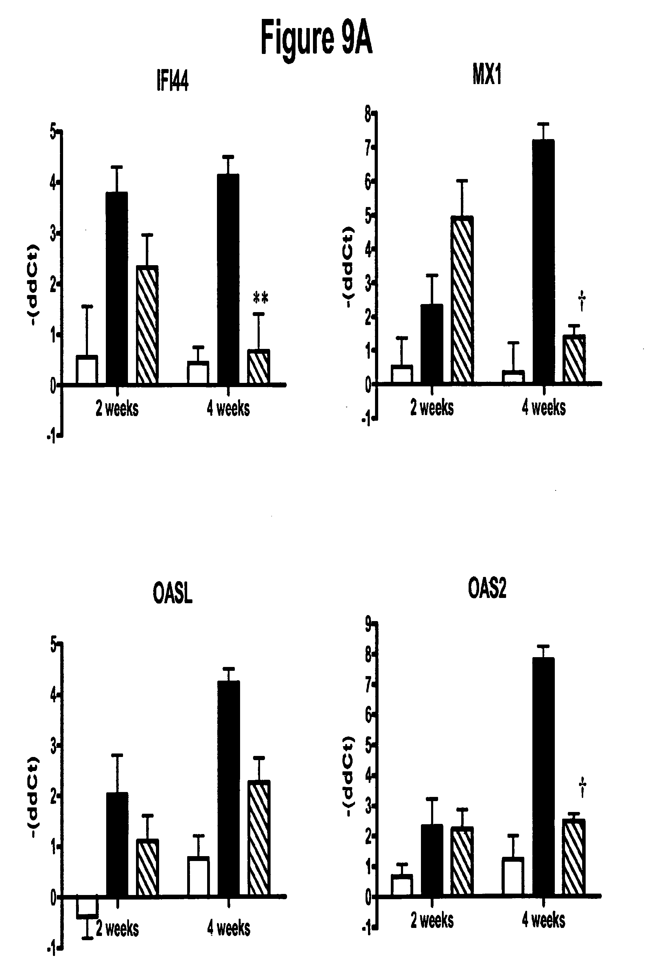

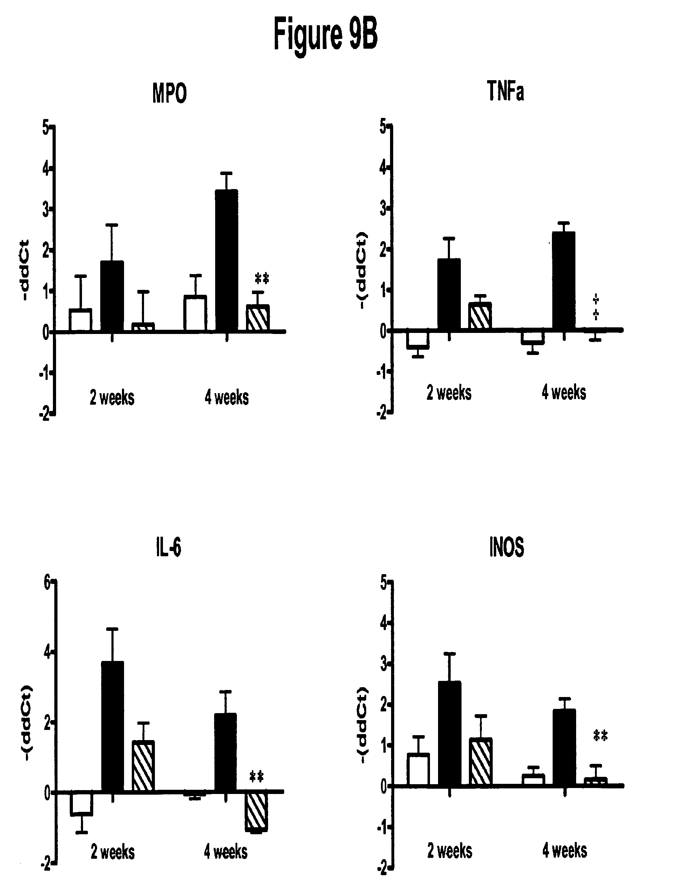

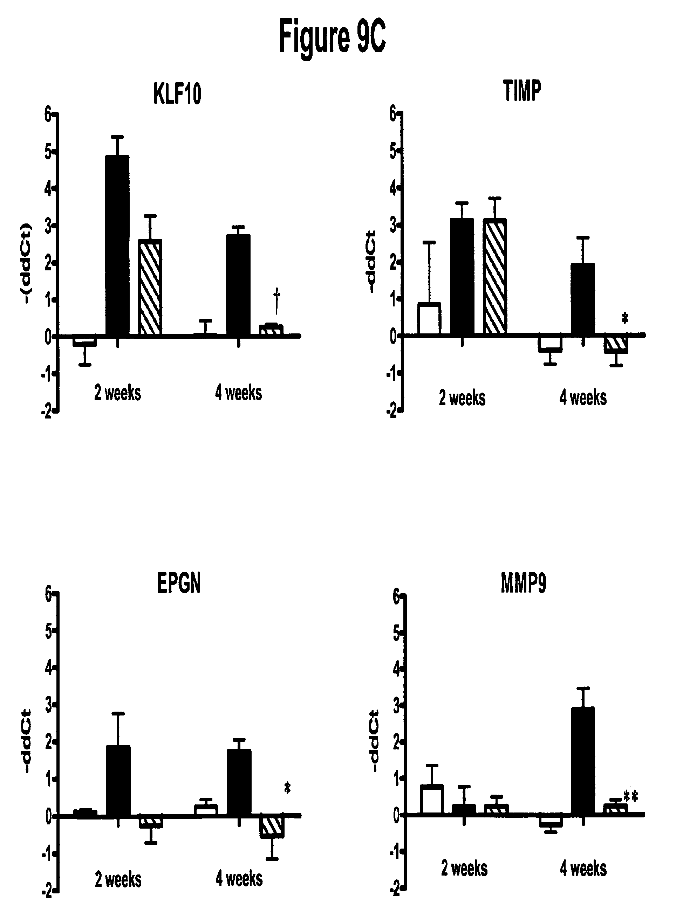

**Figures 9A - 9C** summarize data from the GVH-induced SSc animal model. Fluidigm (qPCR) analysis was performed on skin samples from mice treated twice weekly with 10 mg/kg of body weight of the anti-IFNAR1 murine antibody 5A3 (hatched bars) or control Ig (solid bars), and compared to non-SSc skin (open bars). Figure 9A summarizes the results of expression of four IFN-inducible genes (IFI44, MX1, OASL, OAS2) at two timepoints, and the data indicates that early expression is IFNAR1-independent and that chronic expression is IFNAR-1-dependent. Figure 9B summarizes results demonstrating that inflammatory gene expression (MPO, TNFα, IL-6, INOS) in skin is reduced with anti-IFNAR1 antibody treatment. Figure 9C summarizes results demonstrating that tissue remodeling-related gene expression (KLF10, TIMP, EPGN, MMP9) is reduced with anti-IFNAR1 antibody treatment.

### DETAILED DESCRIPTION OF THE INVENTION

### SCLERODERMA THERAPY

The present invention provides an antagonist of type I IFN for use in methods of treating scleroderma or systemic sclerosis as well as an antagonist of type I IFN for use in methods of treating the symptoms of scleroderma or systemic sclerosis, wherein said antagonist is an anti-IFNaR antibody or an anti-IFNa antibody.

A "therapeutically effective amount" or "therapeutically effective dose" of an anti-type I IFN or anti-interferon alpha or anti-IFNαR antibody of the invention preferably results in a decrease in severity of disease symptoms, an increase in frequency and duration of disease symptom-free periods, or a prevention of impairment or disability due to the disease affliction. In the case of, for example, scleroderma, a therapeutically effective amount or dose preferably prevents further deterioration of physical symptoms associated with scleroderma or systemic sclerosis, such as, for example, dermal fibrosis, skin lesions, alopecia, inflammation, dermal thickening, collagen deposition, proteinuria, autoantibody production, and complement deposition. A therapeutically effective amount or dose preferably also prevents or delays onset of scleroderma or systemic sclerosis, such as may be desired when early or preliminary signs of the disease are present. Likewise it includes delaying chronic progression associated with scleroderma or systemic sclerosis. Laboratory tests utilized in the diagnosis of scleroderma or systemic sclerosis include chemistries, hematology, histopathology, serology and radiology. Accordingly, any clinical or biochemical assay that monitors any of the foregoing may be used to determine whether a particular treatment is a therapeutically effective dose for treating scleroderma or systemic sclerosis. One of ordinary skill in the art would be able to determine such amounts based on such factors as the subject's size, the severity of the subject's symptoms, and the particular composition or route of administration selected.

As used herein, the term "treating" refers to alleviating, ameliorating, and/or decreasing the severity of scleroderma or systemic sclerosis and associated symptoms.

In some embodiments, an antagonist of type I IFN is provided for use in methods of treating one or more of the symptoms of scleroderma, which include dermal fibrosis, skin lesions, alopecia, inflammation, dermal thickening, collagen deposition, proteinuria, autoantibody production, and complement deposition.

The severity, progression, response to treatment, and other clinical measures of the symptoms of scleroderma typically include an evaluation of the patient using the modified Rodnan skin score, the Raynaud's Condition Score, measurements of the forced vital capacity as part of pulmonary function tests, right heart catheterization haemodynamics, measurements of serum creatine, blood pressure and complete blood counts, and measurements of serum creatinine phosphokinase levels (see, for example, Furst, 2008, Rheumatology, 47:v29-v30 and Furst et al., 2007, J. of Rheumatology, 34:5, 1194-1200).

The modified Rodnan skin score is a clinical scoring of scleroderma using established palpation to estimate skin thickness in a patient, using measurements from seventeen skin sites on the patient and grading each site with a 0 = normal skin, 1 = thickened skin, 2 = thickened skin and unable to pinch, and 3 = thickened skin and unable to move, for a total of 51 points (see Czirjak et al., 2007, Ann Rheum Dis.; 66(7):966-9. Epub 2007 Jan 18 and Brennan et al., 1992, Br J Rheumatol.; 31(7):457-60).

Accordingly, in certain embodiments, an antagonist of type I IFN for use in methods of treating scleroderma in a patient in need of such treatment is provided, wherein a therapeutically effective amount of an antagonist of type I interferon is administered, wherein said treating results in an improvement in symptoms as measured by the modified Rodnan skin score (*i.e.* a reduction in the total modified skin score value).

In one embodiment, the invention provides an antagonist of type I IFN for use in a method of treating scleroderma in a patient in need of such treatment, wherein a therapeutically effective amount of an antagonist of type I interferon is administered, wherein said patient has a pre-treatment modified Rodnan skin score of 1 to 51 and a post-treatment modified Rodnan skin score of (1 to 51)-x, where x = 1 to 51 and the post-treatment modified Rodnan skin score is not below 0.

In one embodiment, the invention provides a an antagonist of type I IFN for use in a method of treating scleroderma in a patient in need of such treatment, wherein a therapeutically effective amount of an antagonist of type I interferon is administered, wherein said patient has a pre-treatment modified Rodnan skin score of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or 51, and a post-treatment modified Rodnan skin score of 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, or 0.

In one embodiment, the invention provides an antagonist of type I IFN for use in a method of treating scleroderma in a patient in need of such treatment, wherein a therapeutically effective amount of an antagonist of type I interferon is administered, wherein said treating reduces the modified Rodnan skin score value by at least 1. In one embodiment, the invention provides an antagonist of type I IFN for use in a method of treating scleroderma in a patient in need of such treatment, wherein a therapeutically effective amount of an antagonist of type I interferon is administered, wherein said treating reduces the modified Rodnan skin score value by at least 5. In one embodiment, the invention provides an antagonist of type I IFN for use in a method of treating scleroderma in a patient in need of such treatment, wherein a therapeutically effective amount of an antagonist of type I interferon is administered, wherein said treating reduces the modified Rodnan skin score value by at least 10. In one embodiment, the invention provides an antagonist of type I IFN for use in a method of treating scleroderma in a patient in need of such treatment, wherein a therapeutically effective amount of an antagonist of type I interferon is administered, wherein said treating reduces the modified Rodnan skin score value by at least 25.

In one embodiment, the invention provides an antagonist of type I IFN for use in a method of treating scleroderma in a patient in need of such treatment, wherein a therapeutically effective amount of an antagonist of type I interferon is administered, wherein said treating reduces the modified Rodnan skin score value by at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, or 51.

In certain embodiments, an antagonist of type I IFN for use in methods of treating scleroderma in a patient in need of such treatment is provided, wherein a therapeutically effective amount of an antagonist of type I interferon is administered, wherein said treating results in an improvement in symptoms as measured by the Raynaud's Condition Score (RCS). The RCS is a daily self-assessment of Rayaud's Phenomenom activity using a scale of 0-10, with an increasing number indicating a worsening of symptoms associated with scleroderma (see, for example, Merkel et al., 2002, Arthritis & Rheumatism, 46:9, pp 2410-2420).

In one embodiment, the invention provides an antagonist of type I IFN for use in a method of treating scleroderma in a patient in need of such treatment, wherein a therapeutically effective amount of an antagonist of type I interferon is administered, wherein said patient has a pre-treatment RCS score of 1 to 10 and a post-treatment RCS score of (1 to 10)-x, where x = 1 to 10 and the post-treatment RCS score is not below 0.

In one embodiment, the invention provides an antagonist of type I IFN for use in a method of treating scleroderma in a patient in need of such treatment, wherein a therapeutically effective amount of an antagonist of type I interferon is administered, wherein said patient has a pre-treatment RCS score of 1, 2, 3, 4, 5, 6, 7, 8, 9, or 10, and a post-treatment modified Rodnan skin score of 9, 8, 7, 6, 5, 4, 3, 2, 1, or 0.

The invention provides an antagonist of type I IFN for use in the following disclosed methods.

Disclosed herein is a method of treating scleroderma in a patient in need of such treatment, comprising administering a therapeutically effective amount of an antagonist of type I interferon, wherein said treating reduces the RCS score value by at least 1. Also disclosed herein is a method of treating scleroderma in a patient in need of such treatment, comprising administering a therapeutically effective amount of an antagonist of type I interferon, wherein said treating reduces the RCS score value by at least 2. Also disclosed herein is a method of treating scleroderma in a patient in need of such treatment, comprising administering a therapeutically effective amount of an antagonist of type I interferon, wherein said treating reduces the RCS score value by at least 3. Also disclosed herein is a method of treating scleroderma in a patient in need of such treatment, comprising administering a therapeutically effective amount of an antagonist of type I interferon, wherein said treating reduces the RCS score value by at least 5. Also disclosed herein is a method of treating scleroderma in a patient in need of such treatment, comprising administering a therapeutically effective amount of an antagonist of type I interferon, wherein said treating reduces the RCS score value by at least 6. Also disclosed herein is a method of treating scleroderma in a patient in need of such treatment, comprising administering a therapeutically effective amount of an antagonist of type I interferon, wherein said treating reduces the RCS score value by at least 7. Also disclosed herein is a method of treating scleroderma in a patient in need of such treatment, comprising administering a therapeutically effective amount of an antagonist of type I interferon, wherein said treating reduces the RCS score value by at least 8. Also disclosed herein is a method of treating scleroderma in a patient in need of such treatment, comprising administering a therapeutically effective amount of an antagonist of type I interferon, wherein said treating reduces the RCS score value by at least 9. Also disclosed herein is a method of treating scleroderma in a patient in need of such treatment, comprising administering a therapeutically effective amount of an antagonist of type I interferon, wherein said treating reduces the RCS score value by 10.

Also disclosed herein is a method of treating scleroderma in a patient in need of such treatment, comprising administering a therapeutically effective amount of an antagonist of type I interferon, wherein said treating reduces the RCS score value by at least 1, at least 2, at least 3, at least 4, at least 5, at least 6, at least 7, at least 8, at least 9, or 10.

Also disclosed herein are methods of treating scleroderma in a patient in need of such treatment, comprising administering a therapeutically effective amount of an antagonist of type I interferon, wherein said treating results in an improvement in symptoms as measured by clinical measurements of serum creatinine.

Also disclosed herein are methods of treating scleroderma in a patient in need of such treatment, comprising administering a therapeutically effective amount of an antagonist of type I interferon, wherein said treating results in an improvement in symptoms as measured by measurements of serum creatine phosphokinase levels.

Also disclosed herein are methods of treating scleroderma in a patient in need of such treatment, comprising administering a therapeutically effective amount of an antagonist of type I interferon, wherein said treating results in an improvement in symptoms as measured by clinical measurements of the forced vital capacity as part of pulmonary function tests.

Also disclosed herein are methods of treating scleroderma in a patient in need of such treatment, comprising administering a therapeutically effective amount of an antagonist of type I interferon, wherein said treating results in an improvement in symptoms as measured by right heart catheterization haemodynamics.

Also disclosed herein are methods of treating scleroderma in a patient in need of such treatment, comprising administering a therapeutically effective amount of an antagonist of type I interferon, wherein said treating results in an improvement in symptoms as measured by blood pressure and complete blood counts.

Also disclosed herein are methods of treating scleroderma in a patient in need of such treatment, comprising administering a therapeutically effective amount of an antagonist of type I interferon, wherein said treating results in an improvement in symptoms as measured by histopathologic analysis of patient skin samples. This improvement can be measured as a reduction in inflammation as represented by inflammatory cell infiltrate in the tissue sample, collagen deposition, or overall thickening of the dermisTreatment with an antagonist of type I interferon can reduce inflammation by 2-fold. Treatment with an antagonist of type I interferon can reduce inflammation by 3-fold. Treatment with an antagonist of type I interferon can reduce inflammation by 5-fold.

Also disclosed herein are methods of treating scleroderma in a patient in need of such treatment, comprising administering a therapeutically effective amount of an antagonist of type I interferon, wherein said treating results in an improvement in symptoms as measured by qPCR analysis performed on patient skin samples. See, for example, International Patent Application Publication WO/08070137A2, entitled Interferon Alpha-Induced Pharmacodynamic Markers.

Also disclosed herein are methods of treating scleroderma in a patient in need of such treatment, comprising administering a therapeutically effective amount of an antagonist of type I interferon, wherein said treating reduces inflammatory gene expression including, but not limited to, MPO, TNFα, IL-6, and INOS. Also disclosed herein are methods of treating scleroderma in a patient in need of such treatment, comprising administering a therapeutically effective amount of an antagonist of type I interferon, wherein pre-treatment said patient exhibits increased inflammatory gene expression including, but not limited to, MPO, TNFα, IL-6, and INOS, and post-treatment said patient exhibits a reduction in inflammatory gene expression including, but not limited to, MPO, TNFα, IL-6, and INOS. Also disclosed herein are methods of treating scleroderma in a patient in need of such treatment, comprising administering a therapeutically effective amount of an antagonist of type I interferon, wherein said treating yields an at least 2-fold, at least 3-fold, at least 4-fold, at least 5-fold, or at least 10-fold reduction in inflammatory gene expression as measured by qPCR. Also disclosed herein are methods of treating scleroderma in a patient in need of such treatment, comprising administering a therapeutically effective amount of an antagonist of type I interferon, wherein said treating yields a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of inflammatory gene expression as measured by qPCR.

Also disclosed herein are methods of treating scleroderma in a patient in need of such treatment, comprising administering a therapeutically effective amount of an antagonist of type I interferon, wherein said treating reduces tissue remodeling-related gene expression including, but not limited to, KLF10, TIMP, EPGN, and MMP9. Also disclosed herein are methods of treating scleroderma in a patient in need of such treatment, comprising administering a therapeutically effective amount of an antagonist of type I interferon, wherein pre-treatment said patient exhibits increased tissue remodeling-related gene expression including, but not limited to, KLF10, TIMP, EPGN, and MMP9, and post-treatment said patient exhibits a reduction in tissue remodeling-related gene expression including, but not limited to, KLF10, TIMP, EPGN, and MMP9. Also disclosed herein are methods of treating scleroderma in a patient in need of such treatment, comprising administering a therapeutically effective amount of an antagonist of type I interferon, wherein said treating yields an at least 2-fold, at least 3-fold, at least 4-fold, at least 5-fold, or at least 10-fold reduction in tissue remodeling-related gene expression as measured by qPCR. Also disclosed herein are methods of treating scleroderma in a patient in need of such treatment, comprising administering a therapeutically effective amount of an antagonist of type I interferon, wherein said treating yields a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of tissue remodeling-related gene expression as measured by qPCR.

Pharmacodynamic (PD) markers can be used in methods of treating patients with a therapeutic agent that binds to and antagonizes type I IFN activity, more particularly, IFNα activity, or IFNαR activity, methods that identify patients as candidates for a therapeutic agent that binds to and antagonizes type I IFN activity, more particularly, IFNα activity, or IFNαR activity, methods of diagnosing a patient as having a disorder associated with increased type I IFN or, more particularly, IFNα levels, or IFNαR activity, methods of monitoring disease progression of a patient receiving treatment with a therapeutic agent that binds to and antagonizes type I IFN activity, more particularly, IFNα activity, or IFNαR activity, and methods of identifying a candidate therapeutic for treating type I IFN- and, more particularly, IFNα -mediated , or IFNAR-mediated, disorders.

In one aspect the present invention provides an antagonist of type I IFN for use in methods of treating patient having a type I IFN-mediated disease or disorder comprising administering an antagonist of type I IFN activity; wherein the patient comprises a type I IFN-inducible PD marker expression profile; and wherein the antagonist neutralizes the type I IFN -inducible PD marker expression profile of the subject. The disease or disorder is scleroderma or systemic sclerosis.

Also disclosed herein are methods of identifying, diagnosing, treating, and monitoring disease progression in patients. Patients include any animal having a type I IFN- or an IFNα-inducible disease, disorder, or condition. The patient may have the disease, disorder, or condition as a result of experimental research, e.g., it may be an experimental model developed for the disease, disorder, or condition. Alternatively, the patient may have the disease, disorder, or condition in the absence of experimental manipulation. Patients include humans, mice, rats, horses, pigs, cats, dogs, and any animal used for research.

Methods of identifying, diagnosing, treating, and monitoring disease progression in patients using type I IFN-inducible or IFNα-inducible PD marker expression profiles and/or using an antagonist that neutralizes the type I IFN-inducible or IFNα-inducible PD marker expression profile of the patient are disclosed in International Patent Application Publication No. WO/08070137A2 entitled "Interferon Alpha-induced Pharmacodynamic Markers,".

An antagonist of type I IFN that binds to and blocks type I IFN or IFNα or IFNαR activity may neutralize a type I IFN or IFNα-inducible profile. Neutralization of the type I IFN or IFNα-inducible profile is a reduction in at least one, at least two, at least three, at least five, at least seven, at least eight, at least ten, at least twelve, at least fifteen, at least twenty, at least twenty five, at least thirty, at least thirty five, at least forty, at least forty five, or at least fifty genes induced by type I IFN or IFNα. The genes induced by type I IFN or IFNα may be any group of genes in disclosed in International Patent Application Publication No. WO/08070137A2 entitled "Interferon Alpha-induced Pharmacodynamic Markers". Neutralization of the type I IFN or IFNα-inducible profile is a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, at least ten, at least twelve, at least fifteen, at least twenty, at least twenty five, at least thirty, at least thirty five, at least forty, at least forty five, or at least fifty genes in any type I IFN or IFNα-inducible profile. Alternatively, neutralization of the type I IFN or IFNα-inducible profile refers to a reduction of expression of type I IFN or IFNα-inducible genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those type I IFN or IFNα-inducible genes in a control sample. The agent may neutralize the type I IFN or IFNα profile at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates, more particularly, antagonizes type I IFN or IFNα activity may further or alternatively neutralize expression of one or more type I IFN or IFNα subtypes. The IFNα or type-I IFN subtypes may include any more than one, more than two, more than three, more than four, more than five, more than six, more than seven, more than eight, more than nine, or more than ten IFNα or type-I IFN subtypes. These subtypes may include IFNα1, IFNα2, IFNα4, IFNα5, IFNα6, IFNα7, IFNα8, IFNα10, IFNα14, IFNα17, IFNα21, IFNβ, or IFNω. These subtypes may include all of IFNα1, IFNα2, IFNα8, and IFNα14. Alternatively, these subtypes may include IFNα1, IFNα2, IFNα4, IFNα5, IFNα8, IFNα10, IFNα21. Neutralization of the IFNα or type-I IFN subtypes may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, at least seven, at least eight, or at least ten of the subtypes. Neutralization of the IFNα or type-I IFN subtypes may be a reduction in expression of IFNα or type-I IFN subtype genes that is within at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of those IFNα or type I IFN subtypes in a control sample. The agent may neutralize the IFNα or type I IFN subtypes at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

The agent that binds to and modulates, more particularly, antagonizes type I IFN or IFNα activity may further or alternatively neutralize expression of IFNα receptors, either IFNAR1 or IFNAR2, or both, or TNFα, or IFNγ, or IFNγ receptors (either IFNGR1, IFNGR2, or both IFNGR1 and IFNGR2). Neutralization of expression of IFNα receptors, either IFNAR1 or IFNAR2, or both, or TNFα, or IFNγ, or IFNγ receptors (either IFNGR1, IFNGR2, or both IFNGR1 and IFNGR2) may be a reduction of at least 2%, at least 3%, at least 4%, at least 5%, at least 7%, at least 8%, at least 10%, at least 15%, at least 25%, at least 30%, at least 35%, at least 40%, at least 45%, at least 50%, at least 60%, at least 70%, at least 75%, at least 80%, or at least 90% of any of the at least one, at least two, at least three, at least five, or at least six of these genes. Neutralization of expression of IFNα receptors, either IFNAR1 or IFNAR2, or TNFα, or IFNγ, or IFNγ receptors (either IFNGR1, IFNGR2, or both IFNGR1 and IFNGR2) is a reduction of expression of at most 50%, at most 45%, at most 40%, at most 35%, at most 30%, at most 25%, at most 20%, at most 15%, at most 10%, at most 5%, at most 4%, at most 3%, at most 2%, or at most 1% of expression levels of these genes in a control sample. The agent may neutralize expression of IFNα receptors IFNAR1 or IFNAR2, or TNFα, or IFNγ, or IFNγ receptors IFNGR1 or IFNGR2 at doses of 0.3 to 30 mg/kg, 0.3 to 10 mg/kg, 0.3 to 3 mg/kg, 0.3 to 1 mg/kg, 1 to 30 mg/kg, 3 to 30 mg/kg, 5 to 30 mg/kg, 10 to 30 mg/kg, 1 to 10 mg/kg, 3 to 10 mg/kg, or 1 to 5 mg/kg.

### Dosing and Administration

The amount of the composition of the invention which will be effective in the treatment, prevention or management of scleroderma / systemic sclerosis and the symptoms of the disease can be determined by standard research techniques. Selection of the preferred effective dose can be determined (*e.g.*, via clinical trials) by a skilled artisan based upon the consideration of several factors that will be known to one of ordinary skill in the art. Such factors include the symptoms involved, the patient's body mass, the patient's immune status and other factors known by the skilled artisan to reflect the accuracy of administered pharmaceutical compositions.

The precise dose to be employed in the formulation will also depend on the route of administration, and the seriousness of the scleroderma symptoms displayed, and should be decided according to the judgment of the practitioner and each patient's circumstances. Effective doses may be extrapolated from dose-response curves derived from *in vitro* or animal model test systems.

For antibodies, the dosage administered to a patient is typically 0.1 mg/kg to 100 mg/kg of the patient's body weight. In one embodiment, the dosage administered to a patient is between 0.1 mg/kg and 20 mg/kg of the patient's body weight. In another embodiment, the dosage administered to a patient is between 1 mg/kg and 10 mg/kg of the patient's body weight. In another embodiment, the dosage administered to a patient is between 0.3 mg/kg and 30 mg/kg of the patient's body weight. In another embodiment, the dosage administered to a patient is between 0.3 mg/kg and 3.0 mg/kg. In another embodiment, the dosage administered to a patient is 0.1 mg/kg, 0.3 mg/kg, 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg, 10.0 mg/kg, 11.0 mg/kg, 12.0 mg/kg, 13.0 mg/kg, 14.0 mg/kg, 15.0 mg/kg, 16.0 mg/kg, 17.0 mg/kg, 18.0 mg/kg, 19.0 mg/kg, 20.0 mg/kg, or 25 mg/kg. In a specific embodiment, the dosage administered to a patient is selected from the group consisting of 0.1 mg/kg, 0.3 mg/kg, 1.0 mg/kg, 3.0 mg/kg, 10 mg/kg, and 30 mg/kg of the patient's body weight.

In specific embodiments, dosages of an antibody (optionally in a pharmaceutically acceptable carrier as part of a pharmaceutical composition) are at least about 0.0005, 0.001, 0.05, 0.075, 0.1, 0.25, 0.375, 0.5, 1, 2.5, 5, 10, 20, 37.5, or 50 mg/m2 and/or less than about 500, 475, 450, 425, 400, 375, 350, 325, 300, 275, 250, 225, 200, 175, 150, 125, 100, 75, 60, 50, 37.5, 20, 15, 10, 5, 2.5, 1, 0.5, 0.375, 0.1, 0.075 or 0.01 mg/m². In certain embodiments, the dosage is between about 0.0005 to about 200 mg/m², between about 0.001 and 150 mg/m², between about 0.075 and 125 mg/m², between about 0.375 and 100 mg/m², between about 2.5 and 75 mg/m², between about 10 and 75 mg/m², and between about 20 and 50 mg/m². In related embodiments, the dosage of an anti-IFNα or IFNαR used is at least about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5 mg/kg of body weight of a patient.

In specific embodiments, the dose of an anti-IFNα or anti-IFNαR antibody used is at least about 1 to about 10, about 5 to about 15, about 10 to about 20, or about 15 to about 25 mg/kg of body weight of a patient. In certain embodiments, the dose of an anti-IFNα or IFNαR antibody used is at least about 1 to about 20, about 3 to about 15, or about 5 to about 10 mg/kg of body weight of a patient. In other embodiments, the dose of an anti-IFNα or IFNαR antibody used is at least about 5, about 6, about 7, about 8, about 9, or about 10 mg/kg of body weight of a patient. In certain embodiments, a single dosage unit of the antibody (optionally in a pharmaceutically acceptable carrier as part of a pharmaceutical composition) can be at least about 0.5, about 1, about 2, about 4, about 6, about 8, about 10, about 12, about 14, about 16, about 18, about 20, about 22, about 24, about 26, about 28, about 30, about 32, about 34, about 36, about 38, about 40, about 42, about 44, about 46, about 48, about 50, about 52, about 54, about 56, about 58, about 60, about 62, about 64, about 66, about 68, about 70, about 72, about 74, about 76, about 78, about 80, about 82, about 84, about 86, about 88, about 90, about 92, about 94, about 96, about 98, about 100, about 102, about 104, about 106, about 108, about 110, about 112, about 114, about 116, about 118, about 120, about 122, about 124, about 126, about 128, about 130, about 132, about 134, about 136, about 138, about 140, about 142, about 144, about 146, about 148, about 150, about 152, about 154, about 156, about 158, about 160, about 162, about 164, about 166, about 168, about 170, about 172, about 174, about 176, about 178, about 180, about 182, about 184, about 186, about 188, about 190, about 192, about 194, about 196, about 198, about 200, about 204, about 206, about 208, about 210, about 212, about 214, about 216, about 218, about 220, about 222, about 224, about 226, about 228, about 230, about 232, about 234, about 236, about 238, about 240, about 242, about 244, about 246, about 248, or about 250 micrograms/m2. In other embodiments, dose is up to about 1 g per single dosage unit.

Administration of compositions of the invention to a human patient can be by any route, including but not limited to intravenous, intradermal, transdermal, subcutaneous, intramuscular, inhalation (e.g., via an aerosol), buccal (e.g., sub lingual), topical (i.e., both skin and mucosal surfaces, including airway surfaces), intrathecal, intraarticular, intraplural, intracerebral, intra arterial, intraperitoneal, oral, intralymphatic, intranasal, rectal or vaginal administration, by perfusion through a regional catheter, or by direct intralesional injection. In one embodiment, compositions of the invention are administered by intravenous push or intravenous infusion given over defined period (e.g., 0.5 to 2 hours). Compositions of the invention can be delivered by peristaltic means or in the form of a depot, although the most suitable route in any given case will depend, as is well known in the art, on such factors as the species, age, gender and overall condition of the subject, the nature and severity of the condition being treated and/or on the nature of the particular composition (i.e., dosage, formulation) that is being administered. In particular embodiments, the route of administration is via bolus or continuous infusion over a period of time, once or twice a week. In other particular embodiments, the route of administration is by subcutaneous injection, optionally once or twice weekly. In one embodiment, compositions, and/or methods of the invention are administered on an outpatient basis. In another embodiment, compositions and/or methods of the invention are administered using pre-filled syringes.

In certain embodiments, the dose of a composition comprising an anti-IFNα or IFNαR antibody is measured in units of mg/kg of patient body weight. In other embodiments, the dose of a composition comprising an anti-IFNα or IFNαR antibody is measured in units of mg/kg of patient lean body weight (i.e., body weight minus body fat content). In yet other embodiments, the dose of a composition comprising an anti-IFNα or IFNαR antibody is measured in units of mg/m² of patient body surface area. In yet other embodiments, the dose of a composition comprising an anti-IFNα or IFNαR antibody is measured in units of mg per dose administered to a patient. Any measurement of dose can be used in conjunction with compositions and methods of the invention and dosage units can be converted by means standard in the art.

Those skilled in the art will appreciate that dosages can be selected based on a number of factors including the age, sex, species and condition of the subject (e.g., stage of scleroderma), the desired degree of cellular depletion, the disease to be treated and/or the particular antibody or antigen binding fragment being used and can be determined by one of skill in the art. For example, effective amounts of compositions of the invention may be extrapolated from dose response curves derived in vitro test systems or from animal model (e.g., the cotton rat or monkey) test systems. Models and methods for evaluation of the effects of antibodies are known in the art (Wooldridge et al., Blood, 89(8): 2994 2998 (1997)).

Examples of dosing regimens that can be used in methods of the invention include, but are not limited to, daily, three times weekly (intermittent), weekly, or every 14 days. In certain embodiments, dosing regimens include, but are not limited to, monthly dosing or dosing every 6-8 weeks, or weekly for a defined period of time. In one embodiment, dosing can take place weekly for 1, 2, 3, 4, 5, 6, 7,8, 9, 10, 11, or 12 weeks.

Those skilled in the art will appreciate that dosages are generally higher and/or frequency of administration greater for initial treatment as compared with maintenance regimens. All of the above doses are exemplary and can be used in conjunction with compositions and methods of the invention, however where an an anti-IFNα or IFNαR antibody is used in conjunction with an anti-inflammatory agent the lower doses described above may be used.

In certain embodiments, the dosage and rate of delivery can be adjusted and/or the infusion rate can be reduced based on patient's immunogenic response to compositions and methods of the invention. According to another aspect of the invention, a patient may be pretreated with compositions and methods of the invention to detect, minimize immunogenic response, or minimize adverse effects of compositions and methods disclosed herein.

### TYPE I INTERFERON ANTAGONISTS

As used herein, the term "antagonist of type I IFN" refers to any anti-IFNαR antibody or anti-IFNα antibody that blocks, inhibits, suppresses, neutralizes, decreases or otherwise interferes with or abrogates signaling through and/or activation of the interferon alpha receptor (IFNAR). Antagonists of type I IFN may act by interfering with the interaction between any type I IFN and IFNAR. Thus antagonists of type I IFN may act by binding and antagonizing a type I IFN or by binding an antagonizing the IFNAR. Antagonists of type I IFN may bind to either the IFNAR 1 chain of the IFNAR or to the IFNAR 2 chain of the IFNAR, or they may bind to epitopes resulting from the combination of the two chains. In particular embodiments, antagonists of type I IFN are antibodies that bind to the IFNAR 1 chain of the IFNAR, such as those disclosed in U.S. Patent Appl. Publ. Nos. 2006/0029601, 2006/0020118, and U.S Patent No. 6,713,609.

In certain embodiments, the disclosure provides antibodies or antigen binding fragments thereof that selectively bind to one or more type 1 interferon or bind to the IFNAR in such a way as to interfere with ligand binding, such as, for example, by competitive, non-competitive or uncompetitive inhibition. Alternative embodiments provide antagonists that interfere with signal transduction by the IFNAR. Still further embodiments provide antagonists that antagonize the downstream effects of type 1 interferons.

An antagonist of type I interferon may be administered to a patient or a patient may be identified as a candidate for administration of an agent or a therapeutic agent. In some embodiments, an antagonist of type I interferon is any molecule that binds to and blocks type I IFN, IFNα, or IFNAR activity.

### Type I Interferon Antagonist Antibodies

Type 1 interferon antagonists include anti-IFNAR antibodies and/or fragments thereof that bind to a type 1 interferon receptor and thereby block the binding of its ligand *(i.e.,* interferon alpha, interferon beta or interferon omega). Alternatively or additionally, type 1 interferon antagonists may be anti-type 1 interferon antibodies and/or fragments thereof that bind to a type 1 interferon (*i.e.*, interferon alpha, interferon beta or interferon omega) and thereby block its binding to its receptor (*i.e.*, IFNαR). Antibody-mediated inhibition of ligand binding may occur through competitive, non-competitive or uncompetitive inhibition. Alternatively, antibody-based antagonists may act by preventing intracellular signaling through the type 1 interferon receptor.

Thus, included within the scope of the present invention, are chimeric, primatized, veneered, humanized, deimmunized and human anti-IFNAR and anti-type 1 interferon antibodies and/or antigen-binding fragments thereof. Suitable antibody antagonists for use in the therapeutic methods of the present invention include monoclonal antibodies such as, for example, non-human, chimeric, primatized, humanized, de-immunized and/or fully human antibodies or antigen binding fragments thereof. Antibody antagonists may further comprise one or more chemical modifications to increase the circulating half-life of the antibody, or antigen binding fragment thereof, such as, for example, crosslinking to polyethylene glycol (*i.e.*, PEGylation).

In one embodiment, the antibody may be specific for any subtype(s) of type I IFN or IFNα. For instance, the antibody may be specific for any one of IFNα1, IFNα2, IFNα4, IFNα5, IFNα6, IFNα7, IFNα8, IFNα10, IFNα14, IFNα17, IFNα21, IFNβ, or IFNω. Alternatively, the antibody may be specific for any two, any three, any four, any five, any six, any seven, any eight, any nine, any ten, any eleven, or any twelve type I IFN of IFNα subtypes. If the antibody is specific for more than one type I IFN subtype, the antibody may be specific for IFNα1, IFNα2, IFNα4, IFNα5, IFNα8, IFNα10, and IFNα21; or it may be specific for IFNα1, IFNα2, IFNα4, IFNα5, IFNα8, and IFNα10; or it may be specific for IFNα1, IFNα2, IFNα4, IFNα5, IFNα8, and IFNα21; or it may be specific for IFNα1, IFNα2, IFNα4, IFNα5, IFNα10, and IFNα21; or any combinations of these subtypes. Antibodies specific for type I IFN or IFNα include MEDI-545, any biologic or antibody other than MEDI-545, antibodies described in U.S. patent applications 11/009,410 filed December 10, 2004 and 11/157,494 filed June 20, 2005, 9F3 (and humanized variants thereof) and other antibodies described in U.S. Patent No. 7,087,726, NK-2 and YOK5/19 (WO 84/03105), LO-22 (U.S. Patent 4,902,618), 144 BS (U.S. Patent 4,885,166), and EBI-1, EBI-2, and EBI-3 (EP 119476).

In one embodiment, the antibody antagonist is MEDI-545. MEDI-545 is a fully human, 147,000 Dalton IgG1k monoclonal antibody (Mab) that binds to multiple interferon-alpha (IFN-a) subtypes. MEDI-545 is made from 100% human protein sequences, thereby making it a fully human monoclonal antibody. Fully human monoclonal antibodies may have advantages over other forms of monoclonal antibodies, such as chimeric and humanized antibodies, as they may have a more favorable safety profile and may be eliminated less rapidly from the human body, thereby possibly reducing the frequency of dosing. MEDI-545 was derived from an IgG4k antibody, 13H5, which was selected based on functional assays as having the most desirable properties for a potential therapeutic agent. 13H5 was subsequently converted to an IgG1 antibody isotype, produced in CHO cells, and selected for further characterization and preclinical development with an initial designation of MDX-1103, now referred to as MEDI-545. See also U.S. Patent Application Publication No. 2007/0014724, U.S. Provisional Application No. 60/909,232 entitled "Antibodies with Decreased Deamidation Profiles," U.S. Provisional Application No. 60/909,117 entitled "Antibody Formulation," International Patent Application Publication No. WO/08070137A2 entitled "Interferon Alpha-induced Pharmacodynamic Markers," and International Patent Application No. WO/08070135A2 entitled "Methods of Treating Systemic Lupus Erythematosus,". In a specific embodiment, the antibody is not MEDI-545.

Antibodies of the invention include, but are not limited to, synthetic antibodies, monoclonal antibodies, polyclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), BiTE molecules, single chain antibodies Fab fragments, F(ab') fragments, disulfide-linked Fvs (sdFv), and anti-idiotypic (anti-Id) antibodies, and epitope-binding fragments of any of the above. In particular, antibodies of the present invention include immunoglobulin molecules and immunologically active portions of immunoglobulin molecules. Furthermore, the antibodies of the invention can be of any isotype. In one embodiment, antibodies of the invention are of the IgG1, IgG2, IgG3 or IgG4 isotype. The antibodies of the invention can be full-length antibodies comprising variable and constant regions, or they can be antigen-binding fragments thereof, such as a single chain antibody, or a Fab or Fab'2 fragment.

The disclosure also provides an immunoconjugate comprising an antibody of the invention, or antigen-binding portion thereof, linked to a therapeutic agent, such as a cytotoxin or a radioactive isotope. The invention also provides a bispecific molecule comprising an antibody, or antigen-binding portion thereof, of the invention, linked to a second functional moiety having a different binding specificity than said antibody, or antigen binding portion thereof.

Compositions comprising an antibody, or antigen-binding portion thereof, or immunoconjugate or bispecific molecule of the invention and a pharmaceutically acceptable carrier are also provided.

Antibodies that bind IFNαR are known in the art. Nonlimiting examples of these antibodies can be found in, for example, U.S. Patent Appl. Publ. No. 2006/0029601. Antibodies that bind type I interferons are known to the art. Nonlimiting examples of these antibodies can be found in, for example, U.S. Provisional Patent Application Nos. 61/006,962 filed on February 8, 2008, 61/034,618 filed on March 7, 2008, and 61/049,970 filed on May 2, 2008, each of which is entitled "Anti-IFNAR1 Antibodies with Reduced Fc Ligand Affinity." Antibodies that bind multiple subtypes of interferon α are known in the art. Nonlimiting examples of these antibodies can be found in, for example, U.S. Patent No. 7,087,726 and U.S. Patent Appl. Publ. No. 2007/0014724.

In certain embodiments, as discussed hereinabove, it may be desirable to alter the activity of specific subtypes, or combinations of subtypes, of interferon α.

In certain embodiments, it may be desirable to alter the half-life of the anti-type I interferon, anti-interferon α antibodies or anti-IFNAR antibodies. In one embodiment, it may be desirable to decrease the *in vivo* half-life of the antibodies. In another embodiment, it may be desirable to increase the *in vivo* half-life of the antibodies. See, for example, U.S. Patent Application Publication No. 2006/0198840 A1.

The antibodies or fragments thereof can be produced by any of numerous well known methods in the art for the generation, synthesis, and production of antibodies, in particular, by chemical synthesis or by recombinant expression techniques. See, for example, Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; International Application No. PCT/GB91/01134; International Publication Nos. WO 90/02809, WO 91/10737, WO 92/01047, WO 92/18619, WO 93/11236, WO 95/15982, WO 95/20401, WO97/13844; U.S. Pat. Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 5,969,108, International Publication No. WO 92/22324; Mullinax et al., 1992, BioTechniques 12(6):864-869; Sawai et al., 1995, AJRI 34:26-34, Better et al., 1988, Science 240:1041-1043, U.S. Pat. Nos. 4,444,887 4,716,111, International Publication Nos. WO 98/46645, WO 98/50433, WO 98/24893, WO98/16654, WO 96/34096, WO 96/33735, WO 91/1074, International Publication Nos. WO 98/24893, WO 96/34096, WO 96/33735, U.S. Pat. Nos. 5,413,923, 5,625,126, 5,633,425, 5,569,825, 5,661,016, 5,545,806, 5,814,318, 5,939,598, Morrison, 1985, Science 229:1202, Oi et al., 1986, BioTechniques 4:214; Gillies et al., 1989, J. Immunol. Methods 125:191-202, U.S. Pat. Nos. 5,807,715, 4,816,567, 4,816,397, 6,311,415, European Patent No. EP 239,400, International Publication No. WO 91/09967, U.S. Pat. Nos. 5,225,539, 5,530,101, 5,585,089, European Patent Nos. EP 592,106, EP 519,596, Padlan, 1991, Molecular Immunology 28(4/5):489-498, Studnicka et al., 1994, Protein Engineering 7(6):805-814, Roguska et al., 1994, Proc. Natl. Acad. Sci. USA 91:969-973), U.S. Pat. No. 5,565,332, U.S. Pat. Nos. 6,407,213, 5,766,886, WO 9317105, Tan et al., J. Immunol. 169:1119-25 (2002), Caldas et al., Protein Eng. 13(5):353-60 (2000), Morea et al., Methods 20(3):267-79 (2000), Baca et al., J. Biol. Chem. 272(16):10678-84 (1997), Roguska et al., Protein Eng. 9(10):895-904 (1996), Couto et al., Cancer Res. 55 (23 Supp):5973s-5977s (1995), Couto et al., Cancer Res. 55(8):1717-22 (1995), Sandhu J S, Gene 150(2):409-10 (1994), and Pedersen et al., J. Mol. Biol. 235(3):959-73 (1994), Queen et al., U.S. Pat. No. 5,585,089; Riechmann et al., 1988, Nature 332:323, Kutmejer et al., 1994, BioTechniques 17:242), International Publication No. WO 86/05807, International Publication No. WO 89/01036, and U.S. Pat. No. 5,122,464, and U.S. Pat. No. 5,807,715.

### Combination Therapy

In some embodiments, a second agent other than the agent that binds to and antagonizes type I IFN activity or, more particularly, IFNα activity may be administered to the patient. Second agents include, but are not limited to non-steroidal anti-inflammatory drugs such as ibuprofen, naproxen, sulindac, diclofenac, piroxicam, ketoprofen, diflunisal, nabumetone, etodolac, and oxaprozin, indomethacin; anti-malarial drugs such as hydroxychloroquine; corticosteroid hormones, such as prednisone, hydrocortisone, methylprednisolone, and dexamethasone; methotrexate; immunosuppressive agents, such as azathioprine and cyclophosphamide; and biologic agents that, *e.g.*, target T cells such as Alefacept and Efalizumab, or target TNFα, such as, Enbrel, Remicade, and Humira.

In other embodiments, a second agent that is used to counteract the negative influence of the scleroderma vascular disease may be used in combination with the antagonists of the invention. For example, calcium channel blockers are reported to help blood flow to the skin and heart; angiotensin converting enzyme inhibitors (ACE) inhibitors reverse the vasospasm of the scleroderma renal crisis; and bosentan (a new endothelin-1 receptor inhibitor) or epoprostenol (prostacyclin) can improve blood flow in the lung. Further, drugs that reverse vasospasm (calcium channel blockers, bosentan, prostacyclin, or nitric oxide) all have the potential to modify the course of the disease. The final outcome of untreated scleroderma vascular disease is occlusion of the vessels by either thrombus formation or advanced fibrosis of the intima. Accordingly, anti-platelet therapy in the form of low-dose aspirin may be used in combination with the antagonists of the invention. Anti-fibrotic agents including, but not limited to, colchicine, para-aminobenzoic acid (PABA), dimethyl sulfoxide, and D-penicillamine may be used in combination with the antagonists of the invention.

It is envisioned that compositions of the present invention comprise one or more type 1 interferon antagonist, such as, for example, anti-type 1 IFN antibodies or fragments thereof, and anti-IFNAR antibodies or fragments thereof.

### Pharmaceutical Compositions

Nonlimiting examples of pharmaceutical compositions comprising anti-interferon α antibodies for use in the present invention can be found in U.S. patent application no. 60/909,117 entitled "Antibody Formulation". Examples of pharmaceutical compositions comprising anti-type I interferon antibodies can be found in Patent Appl. Publ. No. 2006/0029601.

In one embodiment, a formulation is for parenteral administration. In one embodiment, a formulation is an injectable formulation. In one embodiment, a formulation is for intravenous, subcutaneous, or intramuscular administration. In a specific embodiment, a formulation comprises an anti-type I IFN or anti-interferon alpha or anti-IFNαR antibody wherein said formulation is for subcutaneous injection. In a specific embodiment, an anti-type I IFN or anti-interferon alpha or anti-IFNαR antibody is formulated for subcutaneous administration in a pre-filled syringe.

In one embodiment, a formulation is for intravenous administration wherein said formulation comprises between about 20 mg/ml and about 40 mg/ml anti-type I IFN or anti-interferon alpha or anti-IFNαR antibody or a fragment thereof. In a specific embodiment, a formulation of the invention is for intravenous administration wherein said formulation comprises between about 20 mg/ml and about 40 mg/ml of an anti-type I IFN or anti-interferon alpha or anti-IFNαR antibody.

In one embodiment, a formulation is for subcutaneous administration wherein said formulation comprises between about 70 mg/ml and about 250 mg/ml of an anti-type I IFN or anti-interferon alpha or anti-IFNαR antibody or a fragment thereof. In a specific embodiment, a formulation of the invention is for subcutaneous administration wherein said formulation comprises between about 70 mg/ml and about 250 mg/ml of an anti-type I IFN or anti-interferon alpha or anti-IFNαR antibody.

In one embodiment, a formulation is for aerosol administration.

The present disclosure also provides a pharmaceutical unit dosage form suitable for parenteral administration to a human which comprises an anti-type I IFN or anti-interferon alpha or anti-IFNαR antibody formulation in a suitable container. In one embodiment, a pharmaceutical unit dosage comprises an anti-type I IFN or anti-interferon alpha or anti-IFNαR antibody. In one embodiment, a pharmaceutical unit dosage comprises an intravenously, subcutaneously, or intramuscularly delivered of an anti-type I IFN or anti-interferon alpha or anti-IFNαR antibody formulation. In another embodiment, a pharmaceutical unit dosage comprises aerosol delivered anti-type I IFN or anti-interferon alpha or anti-IFNαR antibody formulation. In a specific embodiment, a pharmaceutical unit dosage comprises a subcutaneously delivered anti-type I IFN or anti-interferon alpha or anti-IFNαR antibody formulation. In another embodiment, a pharmaceutical unit dosage comprises an aerosol delivered anti-type I IFN or anti-interferon alpha or anti-IFNαR antibody formulation. In a further embodiment, a pharmaceutical unit dosage comprises an intranasally administered anti-type I IFN or anti-interferon alpha or anti-IFNαR antibody formulation.

In one embodiment, a formulation is provided in a sealed container.

Also disclosed is a kit comprising an antagonist of type I interferon formulation of the invention.

An anti-IFNα or IFNαR antibody composition may be formulated with a pharmaceutically acceptable carrier. The term "pharmaceutically acceptable" means one or more non toxic materials that do not interfere with the effectiveness of the biological activity of the active ingredients. Such preparations may routinely contain salts, buffering agents, preservatives, compatible carriers, and optionally other therapeutic agents. Such pharmaceutically acceptable preparations may also routinely contain compatible solid or liquid fillers, diluents or encapsulating substances which are suitable for administration into a human. When used in medicine, the salts should be pharmaceutically acceptable, but non pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulfuric, nitric, phosphoric, maleic, acetic, salicylic, citric, boric, formic, malonic, succinic, and the like. Also, pharmaceutically acceptable salts can be prepared as alkaline metal or alkaline earth salts, such as sodium, potassium or calcium salts. The term "carrier" denotes an organic or inorganic ingredient, natural or synthetic, with which the active ingredient is combined to facilitate the application. The components of the pharmaceutical compositions also are capable of being co mingled with the antibodies of the present invention, and with each other, in a manner such that there is no interaction which would substantially impair the desired pharmaceutical efficacy.

According to certain aspects of the disclosure, an anti-IFNα or IFNαR antibody compositions can be prepared for storage by mixing the antibody or immunoconjugate having the desired degree of purity with optional physiologically acceptable carriers, excipients or stabilizers (Remington's Pharmaceutical Sciences, 16th edition, Osol, A. Ed. (1999)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3 pentanol; and m cresol); low molecular weight (less than about 10 residues) polypeptide; proteins, such as serum albumin, gelatin, or immunoglobulins; hydrophilic polymers such as polyvinylpyrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt forming counter ions such as sodium; metal complexes (e.g., Zn protein complexes); and/or non ionic surfactants such as TWEEN, PLURONICS™ or polyethylene glycol (PEG).

Anti-IFNα or IFNαR antibody compositions also may contain, optionally, suitable preservatives, such as: benzalkonium chloride; chlorobutanol; parabens and thimerosal.

Anti-IFNα or IFNαR antibody compositions may conveniently be presented in unit dosage form and may be prepared by any of the methods well known in the art of pharmacy. All methods include the step of bringing the active agent into association with a carrier which constitutes one or more accessory ingredients. In general, an anti-IFNα or IFNαR antibody compositions are prepared by uniformly and intimately bringing the active compound into association with a liquid carrier, a finely divided solid carrier, or both, and then, if necessary, shaping the product.

In one embodiment, the compositions are substantially pyrogen free.

Compositions suitable for parenteral administration conveniently comprise a sterile aqueous or non aqueous preparation of an anti-IFNα or IFNαR, which is preferably isotonic with the blood of the recipient. This preparation may be formulated according to known methods using suitable dispersing or wetting agents and suspending agents. The sterile injectable preparation also may be a sterile injectable solution or suspension in a non toxic parenterally acceptable diluent or solvent, for example, as a solution in 1,3 butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di glycerides. In addition, fatty acids such as oleic acid may be used in the preparation of injectables. Carrier formulation suitable for oral, subcutaneous, intravenous, intramuscular, etc. administration can be found in Remington's Pharmaceutical Sciences, Mack Publishing Co., Easton, PA. In certain embodiments, carrier formulation suitable for various routes of administration can be the same or similar to that described for RITUXAN™. See, Physicians' Desk Reference (Medical Economics Company, Inc., Montvale, NJ, 2005), pp. 958 960 and 1354 1357. In certain embodiments of the invention, an anti-IFNα or IFNαR antibody compositions are formulated for intravenous administration with sodium chloride, sodium citrate dihydrate, polysorbate 80, and sterile water where the pH of the composition is adjusted to approximately 6.5. Those of skill in the art are aware that intravenous injection provides a useful mode of administration due to the thoroughness of the circulation in rapidly distributing antibodies. Intravenous administration, however, is subject to limitation by a vascular barrier comprising endothelial cells of the vasculature and the subendothelial matrix. In certain embodiments, an anti-IFNα or IFNαR antibodies of compositions and methods of the invention are self administered subcutaneously. In such embodiments, the composition is formulated as a lyophilized drug or in a liquid buffer (e.g., PBS and/or citrate) at about 50 mg/mL.

The formulation herein may also contain more than one active compound as necessary for the particular indication being treated, preferably those with complementary activities that do not adversely affect each other. For example, it may be desirable to further provide an additional immunosuppressive agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin microcapsule and poly (methylmethacylate) microcapsule, respectively, in colloidal drug delivery systems (for example, liposomes, albumin microspheres, microemulsions, nano particles and nanocapsules) or in macroemulsions. Such techniques are disclosed in Remington's Pharmaceutical Sciences 16th edition, Osol, A. Ed. (1980).

The formulations to be used for in vivo administration are typically sterile. This is readily accomplished by filtration through sterile filtration membranes. In one embodiment, sterile refers to being subtantially pyrogen free.

Sustained release preparations may be prepared. Suitable examples of sustained release preparations include semipermeable matrices of solid hydrophobic polymers containing an an anti-IFNα or IFNαR antibody, which matrices are in the form of shaped articles, e.g., films, or microcapsule. Examples of sustained release matrices include polyesters, hydrogels (for example, poly(2 hydroxyethyl methacrylate), or poly(vinylalcohol)), polylactides (U.S. Patent No. 3,773,919), copolymers of L glutamic acid and γ ethyl L glutamate, non degradable ethylene vinyl acetate, degradable lactic acid glycolic acid copolymers such as the LUPRON DEPOT™ (injectable microspheres composed of lactic acid glycolic acid copolymer and leuprolide acetate), and poly D ( ) 3 hydroxybutyric acid. While polymers such as ethylene vinyl acetate and lactic acid glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devized for stabilization depending on the mechanism involved. For example, if the aggregation mechanism is discovered to be intermolecular S S bond formation through thio disulfide interchange, stabilization may be achieved by modifying sulfhydryl residues, lyophilizing from acidic solutions, controlling moisture content, using appropriate additives, and developing specific polymer matrix compositions. In certain embodiments, the pharmaceutically acceptable carriers used in compositions of the invention do not affect human ADCC or CDC.

Anti-IFNα or IFNαR antibody compositions disclosed herein may also be formulated as immunoliposomes. A "liposome" is a small vesicle composed of various types of lipids, phospholipids and/or surfactant which is useful for delivery of a drug (such as an anti-IFNα or IFNαR antibodies disclosed herein) to a human. The components of the liposome are commonly arranged in a bilayer formation, similar to the lipid arrangement of biological membranes. Liposomes containing antibodies of the invention are prepared by methods known in the art, such as described in Epstein et al., Proc. Natl. Acad. Sci. USA, 82:3688 (1985); Hwang et al., Proc. Natl. Acad. Sci. USA, 77:4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG derivatized phosphatidylethanolamine (PEG PE). Liposomes are extruded through filters of defined pore size to yield liposomes with the desired diameter. The antibody of the present invention can be conjugated to the liposomes as described in Martin et al., J. Biol. Chem., 257:286 288 (1982) via a disulfide interchange reaction. A therapeutic agent can also be contained within the liposome. See, Gabizon et al., J. National Cancer Inst., (19)1484 (1989).

### EXAMPLES

The invention is now described with reference to the following examples. These examples are provided for the purpose of illustration only and the invention should in no way be construed as being limited to these examples.

### Example 1. Induction of graft-versus-host (GVH) systemic sclerosis (SSc) mouse model.

To better understand the role of type I interferons (IFNs) in dermal fibrosis, we used a monoclonal antibody directed against IFNAR-1 to block type I IFN signals in a murine model of systemic sclerosis as described below. We investigated the impact of anti-IFNAR on clinical, histological, serological, and molecular disease endpoints in skin and kidneys to identify potential mechanisms by which IFN signaling may promote dermal fibrosis.

SSc can be induced in mice by transferring into mature B- and T cell- deficient mice spleen cells from congenic mice that do not share minor histocompatibility antigens (miHags). A single cell suspension of splenocytes is isolated from 6-10 week old female B10.D2-Hc¹H^{2d}H2-T18^{c}/nSnJ (B10.D2) mice, and red blood cells lysed by 4 minute incubation with a 0.8% ammonium chloride solution. Leukocytes are pelletted by centrifugation at 200xg, washed extensively with phosphate buffered saline (PBS), and 30 x 10^6 cells are injected via lateral tail vein into recipient host 129S6(B6)-*Rag2^{tm1Fwa}*N12 (RAG2-/-) mice.. For IFN signaling blocking studies, 10 mg/kg anti-IFNAR mAb or isotype control IgGlisadministered 2x/week in 0.1mL PBS vehicle via the intraperitoneal route starting 1 day prior to graft, and tissues are harvested for analysis at 2 and 4 weeks.

### Example 2. Blockade of type I IFN signaling in SSc. Scleroderma clinical scores and presentation.

Prophylactic treatment with 10mpk anti-IFNAR mAb 2/weekly significantly reduced skin lesions in SSc mice (**p<.001). Skin was scored weekly as follows: 0= normal; 1 = lesion <1cm2; 2= lesion 1-2cm2; 3 = lesion > 2cm². Extremities (ear, tail, paws) appearing scaly were given a score of 0.3, for a maximum total score of 3.9 per animal. Results representative of 3 replicate studies are presented in Figure 2A.

SSc-induced proteinuria: Mild proteinuria was observed in both anti-IFNAR and Ig control-treated groups, but not in syngeneic graft recipients (see Fig. 2B).

Clinical presentation of SSc: representative mice from anti-IFNAR and control Ig-treated groups, 4 weeks post-graft, are shown in Fig. 2C. Pervasive and severe lesions and alopecia were evident in all control Ig-treated animals, while only 1/5 anti-IFNAR treated animals developed severe lesions.

### Example 3. Histopathogical analysis of SSc skin.

Skin was harvested 4 weeks post-graft from identical dorsal locations on SSc and control mice and compared to ungrafted RAG2-/- skin samples. 5µm H&E and Masson's trichrome stained sections were scored for inflammation (0 = normal; 1= sparse cellular infiltrate; 2 = moderate infiltrate; 3 = pervasive dermal infiltrate) and collagen deposition (0 = normal, 1 = mild; 2 = moderate; 3 = severe). The inflammation and collagen deposition scores were totaled to yield a histopathology score, with a maximum score of 6. Anti-IFNAR treatment reduced total skin pathology by 75% (p<0.001). The data shown in Figure 3A are from 3 replicate studies combined (n= 20 SSc + anti-IFNAR; n= 18 SSc + Ig control). The anti-IFNAR antibody decreased inflammation and thickened dermis in SSc skin, as can be seen in Figure 3B, which shows representative H&E (left) and Masson's trichrome (right) stains of anti-IFNAR and control Ig-treated SSc skin.

### Example 4. Ig and complement deposition in SSc dermis.

5µM frozen skin sections were fixed in acetone for 10 minutes and incubated for 30 minutes with either goat anti-mouse Ig-FITC (green) or rat anti-mouse C1q-PE polyclonal antibody (red) and then mounted in DAPI (blue). The results are presented in Figures 4A - 4F. Ig and C1q were undetectable in syngeneic graft controls (A and D, respectively), but Ig was strongly detected on both Ig control (B) and anti-IFNAR (C) dermal fibroblasts. C1q deposition was observed on dermal fibroblasts (arrow), epidermis, and other dermal structures in animals treated with Ig control (E) but was undetectable in anti-IFNAR treated skin (F).

### Example 5. Autoantibody production and class switching in SSc animals.

Serum anti-Scl-70 and anti-SSA autoantibodies (IgG, IgA, IgM) were detected by ELISA in SSc animals but not controls; no significant differences in total Ig were observed in anti-IFNAR-treated sera. Results are shown in Figure 5A. We found that Ig class switching is intact in anti-IFNAR treated mice. Results shown in Figure 5B reflect that IgG1 was the predominant class of anti-Scl-70 and anti-SSA autoantibodies, and no defect in class switching was observed following IFN blockade. We also examined the splenic architecture in SSc animals 4 weeks post-graft. The results are shown in Figure 5C. 5um frozen spleen sections were stained for CD45R/B220 (brown), and peanut agglutinin (red) to identify germinal centers (GC). GCs were more frequent in SSc than control spleens, but no significant difference was seen in GC frequency or size between anti-IFNAR and Ig control groups.

### Example 6. Donor pDCs are primary source of type I IFN in GVH-SSc.

We examined SSc-induced plasmacytoid dendritic cell expansion by carrying out quantitative analysis by FACS of splenic pDC (B220+/Gr-11o/CD11c+/CD11b-) numbers in mismatch graft recipients 2 weeks post-graft. Results are shown in Figure 6A. Splenic pDC numbers in syngeneic graft recipients were similar to ungrafted RAG2-/- controls (not shown). The donor pDCs were shown to be the primary source of the type I IFN in the GVH-SSc model, by generating grafts of pDC-depleted donor splenocytes. Results are shown in Figure 6B. Gr-1(+) splenocytes are removed by labeling cells for 30 minutes with a rat anti-mouse anti-Gr1 antibody (clone RB68C5) and then incubating 30 minutes with sheep anti-rat IgG-conjugated magnetic beads according to manufacturer's directions. After confirming removal of Gr-11o cells by FACS, remaining splenocytes are grafted into RAG2-/- recipients (30 x 10⁶ cells/mouse). Both total splenocyte and Gr-1(-) graft hosts developed mild proteinuria within 4 weeks, but only the total splenocyte grafts induced skin lesions in host mice (**p<0.01; ***p< 0.001).

### Example 7. Heatmap representation of overexpressed genes in skin that were suppressed by anti-IFNAR mAb treatment.

Whole genome array (WGA) data analysis found that the expression of 308 probe sets that were upregulated by at least 2-fold (p<0.05) in the Ig isotype control Ab-treated group, and were neutralized by anti-IFNAR mAb treatment by at least 50% in skin. Results are depicted in Figure 7. Cell adhesion, onconstatin M signaling via MAPK or Jak/Stat, CCR3 are among the most significant pathways activated in skin and neutralized by IFNAR Ab. 10 probe sets were upregulated in Ig isotype control Ab-treated group by at least 2 fold with p<0.05, and were neutralized by IFNAR Ab by at least 50% in kidney. The type I IFN-inducible genes suppressed by the anti-IFNAR mAb included RSAD2, Ube216, Ube2S, Nfil3, Lysmd2. The type I IFN-inducible genes were determined previously by *ex vivo* stimulation of healthy human donor whole blood with type I IFN family members. Of the pathways suppressed by anti-IFNAR mAb treatment were those involved in cell adhesion, inflammation, cytoskeleton remodeling and apoptosis. By contrast, limited effect of anti-IFNAR mAb treatment was observed in SSc kidneys as compared to that of control Ig treatment (not shown). All samples were profiled using Affymetrix mouse genome 430v2.0 arrays. Hierarchical clustering analyses were performed with SpotFire (http://www.spotfire.com/) and Pathway and network analyses of gene expression data were conducted with the MetaCoreTM integrated software suite from GeneGo, Inc. (St. Joseph, MI).

### Example 8. Real time PCR quantitation of SSc gene expression following IFNAR1 blockade.

The GVH iduced SSc mouse model as described in Example 1 was used to determine expression of various genes associated with inflammation and tissue remodeling. Results of these experiments are summarized in Figures 9A - 9C. Dorsal skin samples were snap frozen and cDNAs generated by reverse transcription of purified mRNAs. Probes specific for the IFN-inducible genes IFI44, MX1, OASL, and OAS2, inflammatory genes MPO, TNFa, IL-6, and iNOS, and remodeling genes KLF10, TIMP, EPGN, and MMP were plated on a Biomark 48.48 Dynamic Array chip (Fluidigm Corp.), and cDNAs were assayed for gene expression. The IFNAR1-blocking mAb 5A3 significantly inhibited induction of all four IFN-inducible genes at the 4 week timepoint (IFI44 by 93%, P<0.006; MX1 by 85%, P<0.0001; OASL by 57%, P<0.02; OAS2 by 81% P<0.0001), but did not significantly impact expression at 2 weeks. Similar results were shown in pro-inflammatory gene induction, where 5A3 completely neutralized MPO, TNFa, IL-6 and iNOS expression at 4 weeks, although a trend toward inhibition at 2 weeks that did not reach statistical significance was also observed. Finally, 5A3 treatment reduced induction of KLF10, a TGF-beta responsive gene, by 47% (P<0.06) at 2 weeks and by 91% (P<0.0001) at 4 weeks, indicating strong inhibition of a major fibrosis pathway. EPGN, an epithelial cell mitogen, was also neutralized >95% (P<0.03) at both timepoints, while the matrix homeostasis-related genes TIMP and MMP9 were significantly inhibited by 5A3 at 4 weeks (by 100% , P<0.05 and 92%, P<0.03, respectively). Taken together, these data demonstrate that, in addition to inhibiting dermal inflammation, IFNAR1 blockade has a dramatic impact on epithelial and fibroblast remodeling in GVH-SSc

### Summary

Our studies demonstrate that type I IFN signaling plays a significant role in dermal fibrosis in a murine model of systemic sclerosis. Antagonizing IFN signaling through IFNαR significantly reduced both the incidence and severity of skin inflammation and dermal remodeling, while proteinuria and scleroderma-associated autoantibody levels were equivalent to that seen in disease controls. Supporting our antibody data was the observation that prior depletion of pDC-inclusive Gr-1(+) populations from donor splenocytes failed to induce skin lesions in host mice, yet did not significantly affect proteinuria.

Whereas, particular embodiments of the invention have been described above for purposes of description, it will be appreciated by those skilled in the art that numerous variations of the details may be made without departing from the invention as defined in the appended claims.

## Claims

1. An antagonist of type I interferon (IFN) for use in a method of treating scleroderma in a patient in need of such treatment wherein said antagonist is an anti-IFNαR antibody or an anti-IFNα antibody.

2. An antagonist of type I interferon for use in a method of alleviating one or more of the symptoms associated with scleroderma in a patient in need of such treatment wherein said antagonist is an anti-IFNαR antibody or an anti-IFNα antibody.

3. The antibody for use of claim 1 or 2, wherein the symptoms of scleroderma are selected from the group consisting of: dermal fibrosis, skin lesions, alopecia, inflammation, dermal thickening, collagen deposition, proteinuria, and complement deposition.

4. The antibody for use of any one of claims 1 to 3, wherein the antibody is administered at a dose between 0.03 mg/kg and 30 mg/kg.

5. The antibody for use of any one of claims 1 to 4, wherein said use in a method of treating or alleviating results in an improvement in symptoms as measured by the modified Rodnan skin score.

6. The antibody for use of any one of claims 1 to 4, wherein said use in a method of treating or alleviating results in an improvement in symptoms as measured by the Raynaud's Condition Score (RCS).

7. The antibody for use of any one of claims 1 to 4, wherein said use in a method of treating or alleviating results in an improvement in symptoms as measured by qPCR analysis performed on patient skin samples.

8. The antibody for use of claim 7, wherein said use in a method of treating and alleviating reduces expression of inflammatory genes selected from the group consisting of MPO, TNFα, IL-6, and INOS.

9. The antibody for use of claim 7, wherein said use in a method of treating or alleviating reduces expression of tissue remodeling-related genes selected from the group consisting of KLF10, TIMP, EPGN, and MMP9.

10. A composition comprising an anti-IFNαR antibody or an anti-IFNα antibody and a pharmaceutically acceptable carrier for use in a method of treating scleroderma in a patient in need of such treatment according to any preceding claim.

11. A composition comprising an anti-IFNαR antibody or an anti-IFNα antibody and a pharmaceutically acceptable carrier for use in a method of alleviating one or more of the symptoms associated with scleroderma in a patient in need of such treatment according to any preceding claim.

12. The composition for use of claim 10 or 11, wherein the composition comprises one or more type I interferon antagonist, optionally wherein the composition comprises an anti-IFNαR antibody and an anti-IFNα antibody.

13. The antibody for use of any one of claims 1 to 9 or the composition for use of any one of claims 10 to 12, wherein a second agent is also administered.

14. The antibody for use or the composition for use of claim 13, wherein the second agent is a non-steroidal anti-inflammatory drug such as ibuprofen, naproxen, sulindac, diclofenac, piroxicam, ketoprofen, diflunisal, nabumetone, etodolac, and oxaprozin, indomethacin; an anti-malarial drug such as hydroxychloroquine; a corticosteroid hormone, such as prednisone, hydrocortisone, methylprednisolone, and dexamethasone; methotrexate; an immunosuppressive agent, such as azathioprine and cyclophosphamide; or a biologic agent that, *e.g.*, targets T cells, such as Alefacept and Efalizumab, or targets TNFα, such as, etanercept, infliximab, and adalimumab.

## Patentansprüche

1. Antagonist von Typ-I-Interferon (IFN) zur Verwendung bei einem Verfahren zur Behandlung von Sklerodermie bei einem Patienten, der einer solchen Behandlung bedarf, wobei es sich bei dem Antagonisten um einen Anti-IFNαR-Antikörper oder einen Anti-IFNα-Antikörper handelt.

2. Antagonist von Typ-I-Interferon zur Verwendung bei einem Verfahren zur Linderung eines oder mehrerer der mit Sklerodermie assoziierten Symptome bei einem Patienten, der einer solchen Behandlung bedarf, wobei es sich bei dem Antagonisten um einen Anti-IFNαR-Antikörper oder einen Anti-IFNa-Antikörper handelt.

3. Antikörper zur Verwendung nach Anspruch 1 oder 2, wobei die Symptome von Sklerodermie aus der aus dermaler Fibrose, Hautläsionen, Alopezie, Entzündung, Hautverdickung, Collagenablagerung, Proteinurie und Komplementablagerung bestehenden Gruppe ausgewählt sind.

4. Antikörper zur Verwendung nach einem der Ansprüche 1 bis 3, wobei der Antikörper in einer Dosis zwischen 0,03 mg/kg und 30 mg/kg verabreicht wird.

5. Antikörper zur Verwendung nach einem der Ansprüche 1 bis 4, wobei die Verwendung bei einem Verfahren zur Behandlung oder Linderung eine Verbesserung der Symptome herbeiführt, wie anhand des Modified Rodnan Skin Score gemessen.

6. Antikörper zur Verwendung nach einem der Ansprüche 1 bis 4, wobei die Verwendung bei einem Verfahren zur Behandlung oder Linderung eine Verbesserung der Symptome herbeiführt, wie anhand des RCS (Raynaud's Condition Score) gemessen.

7. Antikörper zur Verwendung nach einem der Ansprüche 1 bis 4, wobei die Verwendung bei einem Verfahren zur Behandlung oder Linderung eine Verbesserung der Symptome herbeiführt, wie anhand einer an Patientenhautproben durchgeführten qPCR-Analyse gemessen.

8. Antikörper zur Verwendung nach Anspruch 7, wobei durch die Verwendung bei einem Verfahren zur Behandlung oder Linderung die Expression von aus der aus MPO, TNFα, IL-6 und INOS bestehenden Gruppe ausgewählten Entzündungsgenen reduziert wird.

9. Antikörper zur Verwendung nach Anspruch 7, wobei durch die Verwendung bei einem Verfahren zur Behandlung oder Linderung die Expression von aus der aus KLF10, TIMP, EPGN und MMP9 bestehenden Gruppe ausgewählten Genen in Verbindung mit Geweberemodellierung reduziert wird.

10. Zusammensetzung, umfassend einen Anti-IFNαR-Antikörper oder einen Anti-IFNα-Antikörper und einen pharmazeutisch unbedenklichen Trägerstoff zur Verwendung bei einem Verfahren zur Behandlung von Sklerodermie bei einem Patienten, der einer solchen Behandlung bedarf, gemäß einem vorhergehenden Anspruch.

11. Zusammensetzung, umfassend einen Anti-IFNαR-Antikörper oder einen Anti-IFNα.-Antikörper und einen pharmazeutisch unbedenklichen Trägerstoff zur Verwendung bei einem Verfahren zur Linderung eines oder mehrerer der mit Sklerodermie assoziierten Symptome bei einem Patienten, der einer solchen Behandlung bedarf, gemäß einem vorhergehenden Anspruch.

12. Zusammensetzung zur Verwendung nach Anspruch 10 oder 11, wobei die Zusammensetzung einen oder mehrere Typ-I-Interferon-Antagonisten umfasst, gegebenenfalls wobei die Zusammensetzung einen Anti-IFNαR-Antikörper und einen Anti-IFNα-Antikörper umfasst.

13. Antikörper zur Verwendung nach einem der Ansprüche 1 bis 9 oder Zusammensetzung zur Verwendung nach einem der Ansprüche 10 bis 12, wobei auch ein zweites Agens verabreicht wird.

14. Antikörper zur Verwendung oder Zusammensetzung zur Verwendung nach Anspruch 13, wobei es sich bei dem zweiten Agens um einen nichtsteroidalen Entzündungshemmer wie Ibuprofen, Naproxen, Sulindac, Diclofenac, Piroxicam, Ketoprofen, Diflunisal, Nabumeton, Etodolac und Oxaprozin, Indomethacin; ein Antimalariamittel wie Hydroxychloroquin; ein Corticosteroidhormon wie Prednison, Hydrocortison, Methylprednisolon und Dexamethason; Methotrexat; ein Immunsuppressivum wie Azathioprin und Cyclophosphamid; oder ein biologisches Agens, das z.B. auf T-Zellen abzielt, wie Alefacept und Efalizumab, oder auf TNFα abzielt, wie Etanercept, Infliximab und Adalimumab, handelt.

## Revendications

1. Antagoniste d'interféron de type I (IFN) pour utilisation dans un procédé de traitement de la sclérodermie chez un patient nécessitant un tel traitement, **caractérisé en ce que** ledit antagoniste est un anticorps anti-IFNαR ou un anticorps anti-IFNα.

2. Antagoniste d'interféron de type I pour utilisation dans un procédé de soulagement d'un ou plusieurs des symptômes associés à la sclérodermie chez un patient nécessitant un tel traitement **caractérisé en ce que** ledit antagoniste est un anticorps anti-IFNαR ou un anticorps anti-IFNα.

3. Anticorps pour utilisation de la revendication 1 ou 2, **caractérisé en ce que** les symptômes de sclérodermie sont choisis dans le groupe constitué de : fibrose dermique, lésions cutanées, alopécie, inflammation, épaississement dermique, dépôt de collagène, protéinurie, et dépôt de complément.

4. Anticorps pour utilisation de l'une quelconque des revendications 1 à 3, **caractérisé en ce que** l'anticorps est administré à une dose comprise entre 0,03 mg/kg et 30 mg/kg.

5. Anticorps pour utilisation de l'une quelconque des revendications 1 à 4, **caractérisé en ce que** ladite utilisation dans un procédé de traitement ou de soulagement conduit à une amélioration des symptômes telle que mesurée par le score cutané de Rodnan modifié.

6. Anticorps pour utilisation de l'une quelconque des revendications 1 à 4, **caractérisé en ce que** ladite utilisation dans un procédé de traitement ou de soulagement conduit à une amélioration des symptômes telle que mesurée par le score de condition de Raynaud (RCS).

7. Anticorps pour utilisation de l'une quelconque des revendications 1 à 4, **caractérisé en ce que** ladite utilisation dans un procédé de traitement ou de soulagement conduit à une amélioration des symptômes telle que mesurée par analyse qPCR effectuée sur des échantillons de peau du patient.

8. Anticorps pour utilisation de la revendication 7, **caractérisé en ce que** ladite utilisation dans un procédé de traitement et de soulagement réduit l'expression de gènes inflammatoires choisis dans le groupe constitué de MPO, TNFα, IL-6, et INOS.

9. Anticorps pour utilisation de la revendication 7, **caractérisé en ce que** ladite utilisation dans un procédé de traitement ou de soulagement réduit l'expression de gènes associés au remodelage tissulaire choisis dans le groupe constitué de KLF10, TIMP, EPGN et MMP9.

10. Composition comprenant un anticorps anti-IFNαR ou un anticorps anti-IFNα et un véhicule pharmaceutiquement acceptable pour utilisation dans un procédé de traitement de la sclérodermie chez un patient nécessitant un tel traitement selon l'une quelconque des revendications précédentes.

11. Composition comprenant un anticorps anti-IFNαR ou un anticorps anti-IFNα et un véhicule pharmaceutiquement acceptable pour utilisation dans un procédé de soulagement d'un ou plusieurs des symptômes associés à la sclérodermie chez un patient nécessitant un tel traitement selon l'une quelconque des revendications précédentes.

12. Composition pour utilisation de la revendication 10 ou 11, **caractérisée en ce que** la composition comprend un ou plusieurs antagonistes d'interféron de type I, facultativement **caractérisée en ce que** la composition comprend un anticorps anti-IFNαR et un anticorps anti-IFNα.

13. Anticorps pour utilisation de l'une quelconque des revendications 1 à 9 ou la composition pour utilisation de l'une quelconque des revendications 10 à 12, **caractérisé en ce qu'**un deuxième agent est également administré.

14. Anticorps pour utilisation ou la composition pour utilisation de la revendication 13, **caractérisé en ce que** le deuxième agent est un médicament antiinflammatoire non stéroïdien tel que l'ibuprofène, le naproxène, le sulindac, le diclofénac, le piroxicam, le kétoprofène, le diflunisal, la nabumétone, l'étodolac, et l'oxaprozine, l'indométhacine ; un médicament antipaludéen tel que l'hydroxychloroquine ; une hormone corticostéroïde, telle que la prednisone, l'hydrocortisone, la méthylprednisolone et la dexaméthasone ; le méthotrexate ; un agent immunosuppresseur, tel que l'azathioprine et le cyclophosphamide ; ou un agent biologique qui, par exemple, cible les lymphocytes T, tel que l'aléfacept et l'éfalizumab, ou cible TNFα, tel que, l'étanercept, l'infliximab et l'adalimumab.
